(19) Europäisches Patentamt
European Patent Office
Office européen des brevets

(11) Veröffentlichungsnummer : **0 129 507**
**B1**

(12) # EUROPÄISCHE PATENTSCHRIFT

(45) Veröffentlichungstag der Patentschrift : **01.02.89**

(21) Anmeldenummer : **84810234.9**

(22) Anmeldetag : **14.05.84**

(51) Int. Cl.⁴ : **C 07 D403/04**, C 07 D213/50, C 07 D213/61, A 01 N 43/40, C 07 D213/64, C 07 D405/04

(54) **Mikrobizide Mittel.**

(30) Priorität : **19.05.83 CH 2729/83**

(43) Veröffentlichungstag der Anmeldung : **27.12.84 Patentblatt 84/52**

(45) Bekanntmachung des Hinweises auf die Patenterteilung : **01.02.89 Patentblatt 89/05**

(84) Benannte Vertragsstaaten : **AT BE CH DE FR IT LI LU SE**

(56) Entgegenhaltungen :
**EP-A- 0 016 974**
**EP-A- 0 062 238**
**EP-A- 0 069 330**
**EP-A- 0 074 018**
**GB-A- 2 015 524**
**Die Akte enthält technische Angaben, die nach dem Eingang der Anmeldung eingereicht wurden und die nicht in dieser Patentschrift enthalten sind.**

(73) Patentinhaber : **CIBA-GEIGY AG**
**Klybeckstrasse 141**
**CH-4002 Basel (CH)**

(72) Erfinder : **Riebli, Peter**
**Bünten 17**
**CH-4446 Buckten (CH)**

**EP 0 129 507 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft die nachstehend definierten Pyridin-Derivate der Formel I sowie deren Säureadditionssalze. Sie betrifft ferner die Herstellung dieser Verbindungen sowie agrochemische Mittel, die als Wirkstoff mindestens eine der Verbindungen der Formel I enthalten ; ferner die Herstellung dieser Mittel und ein Verfahren zur Bekämpfung oder Verhütung eines Befalls von Pflanzen durch phytopathogene Mikroorganismen.

Bei den erfindungsgemässen Verbindungen handelt es sich um solche der allgemeinen Formel I

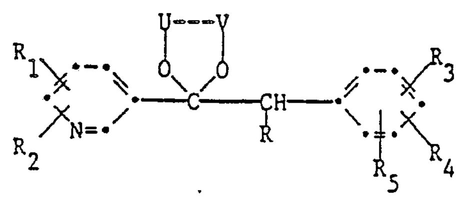

(I)

worin

R für Wasserstoff, $C_1$-$C_6$-Alkyl, $C_3$-$C_6$-Cycloalkyl, Phenyl, Benzyl, durch $C_1$-$C_4$-Alkyl, Halogen, $C_1$-$C_4$-Alkoxy, $NO_2$ und/oder $CF_3$ substituiertes Phenyl oder durch $C_1$-$C_4$-Alkyl, Halogen, $C_1$-$C_4$-Alkoxy, $NO_2$ und/oder $CF_3$ substituiertes Benzyl steht :

$R_1$ und $R_2$ unabhängig voneinander für Wasserstoff, $C_1$-$C_4$-Alkyl, Halogen oder $C_1$-$C_4$-Alkoxy stehen ;

$R_3$, $R_4$ und $R_5$ unabhängig voneinander Wasserstoff, Halogen, $CF_3$, $NO_2$, $C_1$-$C_4$-Alkyl, $C_1$-$C_4$-Alkoxy, Phenyl oder durch $C_1$-$C_4$-Alkyl, $C_1$-$C_4$-Alkoxy, Halogen, $NO_2$ und/oder $CF_3$ substituiertes Phenyl bedeuten ;

U und V unabhängig voneinander für $C_1$-$C_6$-Alkyl oder durch Halogen oder $C_1$-$C_4$-Alkoxy substituiertes Alkyl stehen oder zusammen eine der folgenden Alkylenbrücken

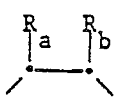 , 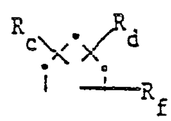 oder 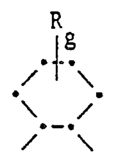 bilden, wobei

$R_a$ für Wasserstoff, $C_1$-$C_{12}$-Alkyl, ein- oder mehrfach durch Halogen substituiertes $C_1$-$C_{12}$-Alkyl, Phenyl, ein- oder mehrfach durch Halogen und/oder $C_1$-$C_4$-Alkyl substituiertes Phenyl oder für die Gruppe $—CH_2—Z—R_h$ steht ;

$R_b$ für $C_1$-$C_{12}$-Alkyl, ein- oder mehrfach durch Halogen substituiertes $C_1$-$C_{12}$-Alkyl, Phenyl, ein- oder mehrfach durch Halogen und/oder $C_1$-$C_4$-Alkyl substituiertes Phenyl oder für die Gruppe $—CH_2—Z—R_h$ steht ;

wobei Z Sauerstoff oder Schwefel bedeutet und

$R_h$ für Wasserstoff, $C_1$-$C_8$-Alkyl, ein durch $C_1$-$C_4$-Alkoxy substituiertes $C_1$-$C_8$-Alkyl, $C_3$-$C_4$-Alkenyl, 2-Propinyl, 3-Halogen-3-propinyl, Phenyl, ein durch Halogen, $C_1$-$C_4$-Alkyl, $C_1$-$C_4$-Alkoxy, $NO_2$ und/oder $CF_3$ substituiertes Phenyl, Benzyl oder durch Halogen, $C_1$-$C_4$-Alkyl, $C_1$-$C_4$-Alkoxy, $NO_2$ und/oder $CF_3$ substituiertes Benzyl steht ;

$R_c$, $R_d$ und $R_f$ unabhängig voneinander Wasserstoff oder $C_1$-$C_4$-Alkyl bedeuten ; und

$R_g$ für Wasserstoff oder $C_1$-$C_4$-Alkyl steht ;

unter Einschluss ihrer Säureadditionssalze.

Unter dem Begriff Alkyl selbst oder als Bestandteil eines anderen Substituenten sind je nach Zahl der angegebenen Kohlenstoffatome beispielsweise folgende Gruppen zu verstehen : Methyl, Ethyl, Propyl, Butyl, Pentyl, Hexyl, Heptyl, Octyl, Nonyl, Decyl, Undecyl oder Dodecyl sowie ihre Isomeren wie z. B. Isopropyl, Isobutyl, tert.-Butyl, sek.-Butyl, Isopentyl usw. Alkenyl steht z. B. für Propenyl-(1), Allyl, Butenyl-(1), Butenyl-(2) oder Butenyl-(3). Halogen soll hier und im folgenden Fluor, Chlor, Brom oder Jod, vorzugsweise Fluor, Chlor oder Brom bedeuten.

Die Erfindung betrifft sowohl die freien Verbindungen der Formel I, als auch deren Additionssalze mit organischen und anorganischen Säuren.

Beispiele salzbildender Säuren sind anorganische Säuren : Halogenwasserstoffsäuren wie Fluorwasserstoffsäure, Chlorwasserstoffsäure, Bromwasserstoffsäure oder Jodwasserstoffsäure sowie Schwefelsäure, Phosphorsäure, phosphorige Säure, Salpetersäure und organische Säuren wie Essigsäure, Trifluoressigsäure, Trichloressigsäure, Propionsäure, Weinsäure, Glycolsäure, Thiocyansäure, Milchsäure, Bernsteinsäure, Zitronensäure, Benzoesäure, Zimtsäure, Oxalsäure, Ameisensäure, Benzolsulfonsäure, p-Toluolsulfonsäure, Methansulfonsäure, Salicylsäure, p-Aminosalicylsäure, 2-Phenoxybenzoesäure oder 2-Acetoxybenzoesäure.

Die Verbindungen der Formel I sind bei Raumtemperatur stabile Oele, Harze oder Feststoffe, die sich durch sehr wertvolle mikrobizide, insbesondere pflanzenfungizide Eigenschaften auszeichnen. Sie lassen sich daher bevorzugt auf dem Agrarsektor oder verwandten Gebieten zur Bekämpfung von phytopathogenen Mikroorganismen einsetzen.

Eine bevorzugte Gruppe von Mikrobiziden, bilden Verbindungen der Formel I, worin R für Wasserstoff oder Methyl steht ; $R_1$ und $R_2$ unabhängig voneinander für Wasserstoff, Methyl, Ethyl, Fluor, Chlor, Brom oder Methoxy stehen ; $R_3$, $R_4$ und $R_5$ unabhängig voneinander für Wasserstoff, Fluor, Chlor, Brom, $CF_3$, $NO_2$, Methyl oder Methoxy stehen ; U und V unabhängig voneinander $C_1$-$C_3$-Alkyl bedeuten oder zusammen folgende Alkylenbrücke

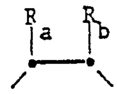

bilden, wobei $R_a$ für Wasserstoff, $C_1$-$C_4$-Alkyl, Phenyl, ein- bis dreifach durch Fluor, Chlor, Brom und/oder Methyl substituiertes Phenyl oder die Gruppe $—CH_2—Z—R_n$ steht ; $R_b$ für $C_1$-$C_4$-Alkyl, ein- bis dreifach durch Fluor, Chlor und/oder Brom substituiertes $C_1$-$C_4$-Alkyl, Phenyl, ein- bis dreifach durch Fluor, Chlor, Brom und/oder Methyl substituiertes Phenyl oder die Gruppe $—CH_2—Z—R_h$ steht ; wobei Z für Sauerstoff oder Schwefel steht ; $R_h$ Wasserstoff, $C_1$-$C_6$-Alkyl, ein durch $C_1$-$C_4$-Alkoxy substituiertes $C_1$-$C_3$-Alkyl, $C_3$-$C_4$-Alkenyl, 2-Propinyl, 2-Halogen-2-propinyl, Phenyl, ein durch Fluor, Chlor, Brom, Methyl, Methoxy, Nitro und/oder $CF_3$ substituiertes Phenyl, Benzyl oder ein durch Fluor, Chlor, Brom, Methyl, Methoxy, $NO_2$ und/oder $CF_3$ substituiertes Benzyl bedeutet ; unter Einschluss ihrer Säureadditionssalze.

Diese Verbindungen sollen hier und im folgenden als Untergruppe Ia bezeichnet werden.

Eine andere bevorzugte Gruppe von Mikrobiziden, bilden Verbindungen der Formel I, worin R für Wasserstoff oder Methyl steht ; $R_1$ und $R_2$ unabhängig voneinander für Wasserstoff, Methyl, Ethyl, Fluor, Chlor, Brom oder Methoxy stehen ; $R_3$, $R_4$ und $R_5$ unabhängig voneinander für Wasserstoff, Fluor, Chlor, Brom, $CF_3$, $NO_2$, Methyl oder Methoxy stehen ; U und V unabhängig voneinander $C_1$-$C_3$-Alkyl bedeuten oder zusammen folgende Alkylenbrücke

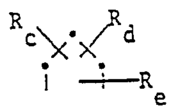

bilden wobei $R_c$, $R_d$ und $R_e$ unabhängig voneinander für Wasserstoff oder $C_1$-$C_4$-Alkyl stehen und die Summe der Kohlenstoffatome in $R_c$, $R_d$ und $R_e$ die Zahl 6 nicht übersteigt ; unter Einschluss ihrer Säureadditionssalze.

Diese Untergruppe soll hier und im folgenden die Bezeichnung Ib tragen.

Eine bevorzugte Gruppe von Mikrobiziden, bilden Verbindungen der Formel I, worin R für Wasserstoff oder Methyl steht ; $R_1$ und $R_2$ unabhängig voneinander für Wasserstoff, Methyl, Ethyl, Fluor, Chlor, Brom oder Methoxy stehen ; $R_3$, $R_4$ und $R_5$ unabhängig voneinander für Wasserstoff, Fluor, Chlor, Brom, $CF_3$, $NO_2$, Methyl oder Methoxy stehen ; U und V unabhängig voneinander $C_1$-$C_3$-Alkyl bedeuten oder zusammen folgende Alkylenbrücke

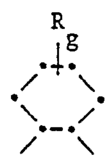

bilden, wobei $R_g$ für Wasserstoff oder $C_1$-$C_4$-Alkyl steht ; unter Einschluss ihrer Säureadditionssalze.

Diese Untergruppe soll hier und im folgenden mit Ic bezeichnet werden.

Eine besonders bevorzugte Gruppe von Mikrobiziden, bilden Verbindungen der Formel I, worin R für Wasserstoff steht ; $R_1$ und $R_2$ unabhängig voneinander für Wasserstoff, Methyl, Fluor, Chlor, Brom oder Methoxy stehen ; $R_3$, $R_4$ und $R_5$ unabhängig voneinander für Wasserstoff, Fluor, Chlor, Brom, $CF_3$, $NO_2$, Methyl oder Methoxy stehen ; U und V zusammen folgende Alkylenbrücke

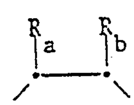

bilden, wobei $R_a$ für Wasserstoff oder Methyl steht ; $R_b$ für $C_1$-$C_4$-Alkyl, durch Fluor, Chlor oder Brom substituiertes $C_1$-$C_4$-Alkyl, Phenyl, ein- bis dreifach durch Fluor, Chlor, Brom und/oder Methyl substituiertes Phenyl oder die Gruppe $—CH_2—Z—R_h$ steht ; wobei Z für Sauerstoff oder Schwefel steht ; $R_h$

Wasserstoff, $C_1$-$C_4$-Alkyl, ein durch $C_1$-$C_4$-Alkoxy substituiertes $C_1$-$C_2$-Alkyl, $C_3$-$C_4$-Alkenyl, 2-Propinyl, 3-Halogen-2-propinyl, Phenyl, ein durch Fluor, Chlor, Brom, Methyl, Methoxy, Nitro und/oder $CF_3$ substituiertes Phenyl, Benzyl oder ein durch Fluor, Chlor, Brom, Methyl, Methoxy, Nitro und/oder $CF_3$ substituiertes Benzyl bedeutet.

Diese wichtige Untergruppe soll nunmehr die Bezeichnung Id tragen.

Eine weitere besonders bevorzugte Gruppe von Mikrobiziden, bilden Verbindungen der Formel I, worin R für Wasserstoff steht ; $R_1$ und $R_2$ unabhängig voneinander für Wasserstoff, Methyl, Fluor, Chlor, Brom oder Methoxy stehen ; $R_3$, $R_4$ und $R_5$ unabhängig voneinander für Wasserstoff, Fluor, Chlor, Brom, $CF_3$, $NO_2$, Methyl oder Methoxy stehen ; U und V zusammen folgende Alkylenbrücke

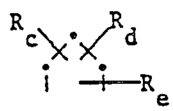

bilden, wobei $R_c$ Wasserstoff bedeutet und $R_d$ und $R_e$ unabhängig voneinander für Wasserstoff, Methyl oder Ethyl stehen.

Diese Untergruppe trägt nunmehr die Bezeichnung Ie.

Eine andere besonders bevorzugte Gruppe von Mikrobiziden, bilden Verbindungen der Formel I, worin R für Wasserstoff Steht ; $R_1$ und $R_2$ unabhängig voneinander für Wasserstoff, Methyl, Fluor, Chlor, Brom oder Methoxy stehen ; $R_3$, $R_4$ und $R_5$ unabhängig voneinander für Wasserstoff, Fluor, Chlor, Brom, $CF_3$, $NO_2$, Methyl oder Methoxy stehen ; U und V zusammen folgende Alkylenbrücke

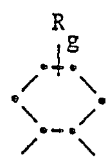

bilden, wobei $R_g$ für Wasserstoff, Methyl oder Ethyl steht.

Diese Untergruppe soll hier und im folgenden die Bezeichnung If tragen.

Auf Grund ihrer ausgeprägten mikrobiziden Wirkung werden z. B. folgende Einzelsubstanzen als Isomerengemische oder in optisch reiner Form ganz besonders bevorzugt :

3-[2-(2,4-Dichlorbenzyl)-4-methoxymethyl-1,3-dioxolan-2-yl]-pyridin,
3-[2-(2,4-Dichlorbenzyl)-4-methyl-1,3-dioxolan-2-yl]-pyridin,
3-[2-(2,4-Dichlorbenzyl)-4-chlormethyl-1,3-dioxolan-2-yl]-pyridin,
3-[2-(2,4-Dichlorbenzyl)-1,3-dioxan-2-yl]-pyridin,
3-[2-(2,4-Dichlorbenzyl)-4-hydroxymethyl-1,3-dioxolan-2-yl]-pyridin,
3-[2-(2,4-Dichlorbenzyl)-4-ethyl-1,3-dioxolan-2-yl]-pyridin.

Die Verbindungen der Formel I können nach einer ganzen Reihe von Reaktionsvarianten A bis E hergestellt werden. Die einzelnen Varianten werden anschliessend eingehend beschrieben. Hierbei haben die Substituenten R, $R_1$, $R_2$, $R_3$, $R_4$, $R_5$, U, V, $R_a$, $R_b$, $R_c$, $R_d$, $R_e$, $R_f$, $R_g$ und $R_h$ in den Ausgangs und Zwischenprodukten die unter Formel I angegebenen Bedeutungen.

Im einzelnen kann man wie folgt vorgehen :

A. Ketale der Formel I können hergestellt werden durch Ketalisierungsreaktion eines Ketons der Formel II in Gegenwart einer Säure

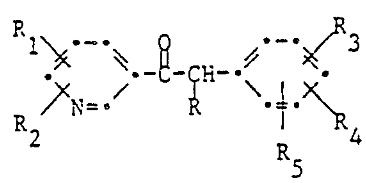 (II)

mit einem Alkohol V—OH oder U—OH, mit einem Diol der Formel HO—U—V—OH oder mit einem Orthocarbonsäureester R'—C(OV)$_3$ oder R'—C(OU)$_3$ oder durch Umketalisierung des erhaltenen Ketals mit einem Ueberschuss des entsprechenden Alkanols oder Diols, dabei haben die Substituenten R bis $R_5$, U und V in Formel II sowie in den Alkoholen und Diolen, die unter Formel I angegebene Bedeutung und R' steht vorzugsweise für einen Niederalkylrest.

Diese Ketalisierungs-Reaktion kann analog zu bereits bekannten Ketalisierungs-Reaktionen durchgeführt werden, beispielsweise analog zur Herstellung von 2-Brommethyl-2,4-diphenyl-1,3-dioxolan [Synthesis, 1974 (I), 23].

Bei der bevorzugten Ausführung der Ketalisierung werden beide Reaktionspartner mehrere Stunden

zusammen mit einem Azeotrop-Bildner in einem der üblichen organischen Lösungsmittel unter Rückfluss erhitzt. Als Azeotrop-Bildner kommen z. B. Benzol, Toluol, Xylol, Chloroform oder Tetrachlorkohlenstoff in Frage, wobei zur Beschleunigung der Reaktion ein Zusatz einer starken Säure, wie z. B. p-Toluolsulfonsäure vorteilhaft sein kann. Verwendbare organische Lösungsmittel sind in diesem Fall z. B. aromatische Kohlenwasserstoffe, wie Benzol, Toluol, Xylol usw. und gesättigte Kohlenwasserstoffe, wie n-Hexan.

B. Speziell, wenn in Verbindungen der Formel I die Substituenten U und V zusammen für —$CH_2$—$CH(CH_2ZR_h)$— stehen, kann man diese durch Reaktion einer Verbindung der Formel III

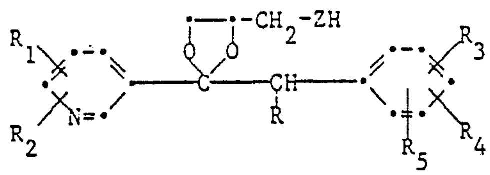

(III)

mit einer reaktionsfähigen, zur O-Alkylierung oder S-Alkylierung geeigneten Verbindung der Formel (IV)

$$R_h—X \quad (IV)$$

herstellen, wobei die Substituenten R bis $R_5$, $R_h$ und Z in den Formeln III und IV, die unter Formel I angegebenen Bedeutungen haben und X für eine der üblichen Abgangsgruppen steht, wie beispielsweise für Halogen, insbesondere Chlor, Brom oder Jod, oder Benzolsulfonyloxy, p-Tosyloxy, Trifluoracetyloxy oder bevorzugt Niederalkylsulfonyloxy wie z. B. Mesyloxy.

Die Reaktion wird bevorzugt in reaktions-inerten organischen Lösungsmitteln durchgeführt. Es eignen sich hierzu z. B. N,N-Dimethylformamid, N,N-Dimethylacetamid, Hexamethylphosphortriamid, Dimethylsulfoxid, 4-Methyl-2-pentanon, usw. Auch Gemische mit anderen reaktionsinerten Lösungsmitteln, z. B. mit aromatischen Kohlenwasserstoffen wie Benzol, Toluol, Xylol, usw. können verwendet werden. In manchen Fällen kann es sich als vorteilhaft erweisen zur Beschleunigung der Reaktionsgeschwindigkeit in Gegenwart einer Base zu arbeiten. Solche Basen sind z. B. Alkalimetallhybride oder Alkalimetallcarbonate. In gewissen Fällen kann es auch von Vorteil sein, dass man die Verbindung III zuerst auf bekannte Art in ein geeignetes Metall-Salz überführt.

Dies geschieht vorzugsweise durch Reaktion von III mit einer Na-Verbindung, z. B. Natriumhydrid, Natriumhydroxid usw. Anschliessend wird dieses Salz von III mit der Verbindung der Formel IV umgesetzt.

Zur Erhöhung der Reaktionsgeschwindigkeit kann in manchen Fällen auch bei erhöhter Temperatur, vorzugsweise 80 °C bis 130 °C, bzw. am Siedepunkt des Lösungsmittels gearbeitet werden.

C. Zur Herstellung von Verbindungen der Formel I, bei denen —U—V— für —$CH_2$—$CH(CH_2$—Z—$R_h)$ steht, kann man auch ein Ketal der Formel V

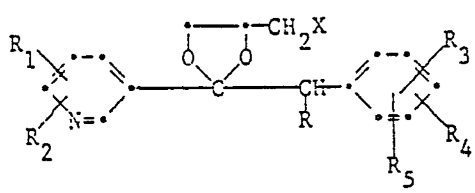

(V)

mit einer Verbindung der Formel VI

$$R_h—Z—M \quad (VI)$$

reagieren lassen ; hierbei sind die Substituenten R bis $R_5$, $R_h$ und Z wie unter Formel I definiert, X hat die in Variante B angegebenen Bedeutungen und M steht für Wasserstoff oder vorzugsweise ein Metall-, insbesondere ein Alkalimetallatom.

D. Bedeutet Z in den Produkten der Formel I Sauerstoff, so sind diese auch durch Kondensations-Reaktion eines Alkohols der Formel VII mit einem Alkanol der Formel VIII zugänglich :

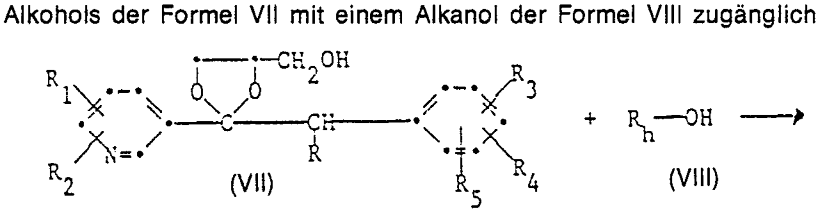

(VII) + $R_h$—OH (VIII) ⟶ (I)

Hierbei sind die Substituenten R bis $R_5$ und $R_h$ wie unter Formel I definiert.

Bei dieser Kondensationsreaktion können die Reaktanden in einem geeigneten Lösungsmittel unter Rückfluss erhitzt werden, wobei gleichzeitig das entstehende Wasser azeotrop aus dem Reaktionsgemisch abdestilliert wird. Als Lösungsmittel kommen aromatische Kohlenwasserstoffe, wie Toluol oder der Alkohol VIII selbst in Frage. Bei dieser Reaktion arbeitet man zweckmässigerweise in Gegenwart einer starken Säure z. B. p-Toluolsulfonsäure.

E. Zur Einführung des Substituenten R in ein unverzweigtes Produkt der Formel I'

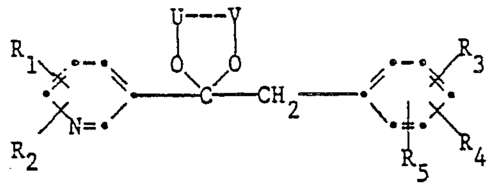

(I')

kann durch Reaktion mit einer Verbindung der Formel IX

$$R—X \qquad (IX)$$

erfolgen, wobei die Substituenten R bis $R_5$ sowie U und V in den Formeln I' und IX wie unter Formel I definiert sind und X für eine übliche, bei Variante B angegebene Abgangsgruppe steht. Diese Reaktion wird zweckmässigerweise unter den in Variante B beschriebenen Bedingungen durchgeführt, wobei man die Verbindung der Formel I' vor der Umsetzung mit IX vorteilhafterweise zuerst in ein Metallsalz, insbesondere ein Alkalisalz überführt. Dies geschieht z. B. analog zu den Umsetzungen von III in Variante B.

Werden die Verbindungen der Formel I als Basen erhalten, dann lassen sie sich durch anorganische oder organische Säuren in entsprechende Salze der Formel I überführen. Umgekehrt lassen sich Salze der Formel I beispielsweise durch Reaktion mit Alkali(hydrogen)carbonat oder Alkalihydroxid in die freien Basen der Formel I überführen.

Die Ausgangsketone der Formel II können nach an sich bekannten Methoden z. B. dadurch hergestellt werden, dass man ein Benzylhalogenid der Formel X

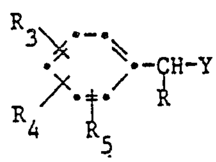

(X)

worin R bis $R_5$ die unter Formel I angegebenen Bedeutungen hat und Y für Halogen, vorzugsweise Chlor, Brom oder Jod bedeutet, mit einem Pyridinderivat der Formel XI,

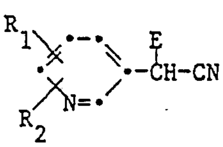

(XI)

worin $R_1$ und $R_2$ die unter Formel I angegebenen Bedeutungen haben und E für einen disubstituierten Aminorest, wie z. B. Dimethylamino, Diethylamino, Piperidino oder Morpholino usw. bedeutet, zu einer Verbindung der Formel XII umsetzt

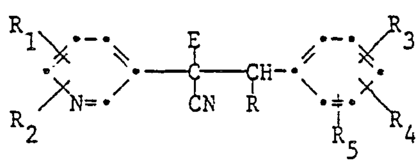

(XII)

und letzteres hydrolysiert.

Die Verbindungen der Formeln V—OH, U—OH, HO—U—V—OH, R'-C(OV)$_3$, R'-C(OU)$_3$, IV, VI, VIII und IX sind allgemein bekannt oder werden nach an sich bekannten Methoden hergestellt.

Die Herstellung der Zwischenprodukte.

Die Ausgangsstoffe der Formeln III, V und VII, die speziell für die Herstellung der erfindungsgemässen Verbindungen der Formel I entwickelt wurden, sind neu und bilden ebenfalls einen Gegenstand der

Erfindung, sie zeigen ebenfalls mikrobizide Eigenschaften.

Die beschriebenen Herstellungsvarianten sind Bestandteil der Erfindung.

Bei allen beschriebenen Ketalisierungs-Reaktionen eines Ketons mit einem substituierten α,β- oder α,γ-Diol entstehen vorwiegend Gemische von Diastereomeren des resultierenden Ketals. Entsprechend bilden sich aus den Ausgangsketonen im allgemeinen Diastereomerengemische der Endprodukte I. Die Verbindungen der Formel I können beispielsweise in nachfolgenden beiden diastereomeren Formen vorliegen :

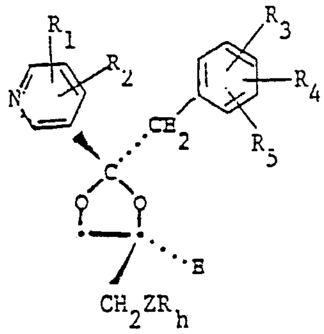

A-Typen

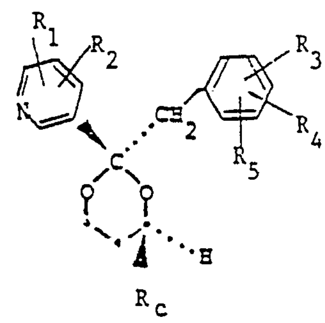

Die Konfiguration vom Typ A soll hier und im folgenden als das « trans »-Isomere bezeichnet werden.

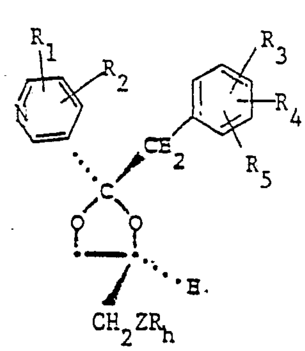

B-Typen

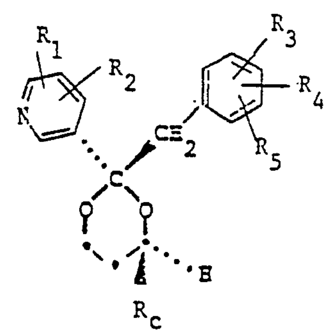

Die Symbole in den räumlich wiedergegebenen Strukturen sollen folgende Bedeutungen haben :

··· = hinter
▬ = in    der Zeichenebene.
► = vor

Die Konfiguration vom Typ B soll entsprechend als das « Cis »-Isomere bezeichnet werden. Die Trennung der beiden Diastereomeren kann beispielsweise durch fraktionierte Kristallisation oder durch Chromatographie (Dünn-, Dickschicht-, Säulenchromatographie, Flüssigkeitshochdruckchromatographie usw.) erfolgen. Die beiden Isomeren zeigen unterschiedliche mikrobizide Wirkung. Im allgemeinen werden für praktische Zwecke die Diastereomerengemische verwendet.

Die Erfindung betrifft sämtliche isomeren Verbindungen der Formel I, ihre Salze und Metallkomplexe.

Das Herstellungsverfahren von Verbindungen der Formel I ist in seinen beschriebenen Varianten A, B, C, D und E Bestandteil der Erfindung.

Einige der in den Verfahren A, B, C, D und E verwendeten Ausgangsstoffe und Zwischenprodukte sind bekannt, andere können nach an sich bekannten Methoden hergestellt werden. Einige sind neu ; ihre Herstellung wird hierin beschrieben.

In der Europaïschen Patentanmeldung Nr. 062238 sind 1,3-Dioxolan-Derivate zur Bekämpfung von phytopathogenen Fungi beschrieben.

Es wurde überraschend festgestellt, dass Verbindungen der Formel I ein für praktische Bedürfnisse sehr günstiges Mikrobizid-Spektrum gegen phytopathogene Pilze und Bakterien aufweisen. Sie besitzen sehr vorteilhafte kurative, präventive und systemische Eigenschaften und lassen sich zum Schutz von Kulturpflanzen einsetzen. Mit den Wirkstoffen der Formel I können an Pflanzen oder an Pflanzenteilen (Früchte, Blüten, Laubwerk, Stengel, Knollen, Wurzeln) von unterschiedlichen Nutzkulturen die auftretenden Mikroorganismen eingedämmt oder vernichtet werden, wobei auch später zuwachsende Pflanzenteile von derartigen Mikroorganismen verschont bleiben.

Die Wirkstoffe sind gegen die den folgenden Klassen angehörenden phytopathogenen Pilze wirksam : Ascomyceten (z. B. Venturia, Podosphaera, Erysiphe, Monilinia, Uncinula) ; Basidiomyceten (z. B. die Gattungen Hemileia, Rhizoctonia, Puccinia) ; Fungi imperfecti (z. B. Botrytis, Helminthosporium,

Fusarium, Septoria, Cercospora und Alternaria). Ueberdies wirken die Verbindungen der Formel I systemisch. Sie können ferner als Beizmittel zur Behandlung von Saatgut (Früchte, Knollen, Körner) und Pflanzenstecklingen zum Schutz vor Pilzinfektionen sowie gegen im Erdboden auftretende phytopathogene Pilze eingesetzt werden. Die erfindungsgemässen Wirkstoffe zeichnen sich durch besonders gute Pflanzenverträglichkeit aus. Ueberraschenderweise zeigen die erfindungsgemässen Verbindungen der Formel I gegenüber den in Dioxolanring unsubstituierten Grundkörpern nicht nur eine bessere Pflanzenverträglichkeit — selbst bei hohen Aufwandmengen —, sondern zusätzlich ein breiteres Wirkungsspektrum und enthalten überdies eine praxisgerechtere Wirkung bei geringer Dosierung.

Die Erfindung betrifft somit auch mikrobizide Mittel sowie die Verwendung der Verbindungen der Formel I zur Bekämpfung phytopathogener Mikroorganismen, insbesondere pflanzenschädigender Pilze bzw. die präventive Verhütung eines Befalls an Pflanzen.

Darüberhinaus schliesst die vorliegende Erfindung auch die Herstellung agrochemischer Mittel ein, die gekennzeichnet ist durch das innige Vermischen der Aktivsubstanz mit einem oder mehreren hierin beschriebenen Substanzen bzw. Substanzgruppen. Eingeschlossen ist auch ein Verfahren zur Behandlung von Pflanzen, das sich durch Applikation der Verbindungen der Formel I bzw. der neuen Mittel auszeichnet.

Als Zielkulturen für die hierin offenbarten Indikationsgebiete gelten im Rahmen dieser Erfindung beispielsweise folgende Pflanzenarten : Getreide : (Weizen, Gerste, Roggen, Hafer, Reis, Sorghum und Verwandte) ; Rüben : (Zucker- und Futterrüben) ; Kern-, Stein- und Beeren obst : (Aepfel, Birnen, Pflaumen, Pfirsiche, Mandeln, Kirschen, Erd-, Him- und Brombeeren) ; Hülsenfrüchte : (Bohnen, Linsen, Erbsen, Soja) ; Oelkulturen : (Raps, Senf, Mohn, Oliven, Sonnenblumen, Kokos, Rizinus, Kakao, Erdnüsse) ; Gurkengewächse : (Kürbis, Gurken, Melonen) ; Fasergewächse : (Baumwolle, Flachs, Hanf, Jute) ; Citrusfrüchte : (Orangen, Zitronen, Pampelmusen, Mandarinen) ; Gemüsesorten : (Spinat, Kopfsalat, Spargel, Kohlarten, Möhren, Zwiebeln, Tomaten, Kartoffeln, Paprika) ; Lorbeergewächse : (Avocado, Cinnamonum, Kampfer) oder Pflanzen wie Mais, Tabak, Nüsse, Kaffee, Zuckerrohr, Tee, Weinreben, Hopfen, Bananen- und Naturkautschukgewächse sowie Zierpflanzen (Compositen). Wirkstoffe der Formel I werden üblicherweise in Form von Zusammensetzungen verwendet und können gleichzeitig oder nacheinander mit weiteren Wirkstoffen auf die zu behandelnde Fläche oder Pflanze gegeben werden. Diese weiteren Wirkstoffe können sowohl Düngemittel, Spurenelement-Vermittler oder andere das Pflanzenwachstum beeinflussende Präparate sein. Es können aber auch selektive Herbizide, Insektizide, Fungizide, Bakterizide, Nematizide, Molluskizide oder Gemische mehrer dieser Präparate sein, zusammen mit gegebenenfalls weiteren in der Formulierungstechnik üblichen Trägerstoffen, Tensiden oder anderen applikationsfördernden Zusätzen.

Geeignete Träger und Zusätze können fest oder flüssig sein und entsprechen den in der Formulierungstechnik zweckdienlichen Stoffen, wie z. B. natürlichen oder regenerierten mineralischen Stoffen, Lösungs-, Dispergier-, Netz-, Haft-, Verdickungs-, Binde- oder Düngemitteln.

Ein bevorzugtes Verfahren zum Aufbringen eines Wirkstoffes der Formel I bzw. eines agrochemischen Mittels, das mindestens einen dieser Wirkstoffe enthält, ist das Aufbringen auf das Blattwerk (Blattapplikation). Anzahl der Applikationen und Aufwandmenge richten sich dabei nach dem Befallsdruck für den entsprechenden Erreger (Pilzsorte). Die Wirkstoffe der Formel I können aber auch über den Erdboden durch das Wurzelwerk in die Pflanze gelangen (systemische Wirkung), indem man den Standort der Pflanze mit einer flüssigen Zubereitung tränkt oder die Substanzen in fester Form in den Boden einbringt z. B. in Form von Granulat (Bodenapplikation). Die Verbindungen der Formel I können aber auch auf Samenkörner aufgebracht werden (Coating), indem man die Körner entweder mit einer flüssigen Zubereitung des Wirkstoffs tränkt oder sie mit einer festen Zubereitung beschichtet. Darüberhinaus sind in besonderen Fällen weitere Applikationsarten möglich, so z. B. die gezielte Behandlung der Pflanzenstengel oder der Knospen.

Die Verbindungen der Formel I werden dabei in unveränderter Form oder vorzugsweise zusammen mit den in der Formulierungstechnik üblichen Hilfsmittel eingesetzt und werden daher z. B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z. B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt. Günstige Aufwandmengen liegen im allgemeinen bei 50 g bis 5 kg Aktivsubstanz (AS) je ha ; bevorzugt 100 g bis 2 kg AS/ha, insbesondere bei 200 g bis 600 g AS/ha.

Die Formulierungen d. h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z. B. durch inniges Vermischen und/oder Vermahlen der Wirkstoffe mit Streckmitteln, wie z. B. mit Lösungsmitteln, festen Trägerstoffen, und gegebenenfalls oberflächenaktiven Verbindungen (Tensiden).

Als Lösungsmittel können in Frage kommen : Aromatische Kohlenwasserstoffe, bevorzugt die Fraktionen $C_8$ bis $C_{12}$, wie z. B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder

Ethylether, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl ; oder Wasser.

Als feste Trägerstoffe, z. B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z. B. Bimsstein, Ziegelbruch, Sepiolit oder Bentonit, als nicht sorptive Trägermaterialien z. B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Besonders vorteilhafte, applikationsfördernde Zuschlagstoffe, die zu einer starken Reduktion der Aufwandmenge führen können, sind ferner natürliche(tierische oder pflanzliche) oder synthetische Phospholipide aus der Reihe der Kephaline und Lecithine, wie z. B. Phosphatidylethanolamin, Phosphatidylserin, Phosphatidylcholin, Sphingomyelin, Phosphatidylinosit, Phosphatidylglycerin, Lysolecithin, Plasmalogene oder Cardiolipin, die man beispielsweise aus tierischen oder pflanzlichen Zellen, insbesondere aus Hirn, Herz, Leber, Eidotter oder Sojabohnen gewinnen kann. Verwendbare Handelsmischungen sind z. B. Phosphatidylcholin-Mischungen. Synthetische Phospholipide sind z. B. Dioctanoylphosphatidylcholin und Dipalmitoylphosphatidylcholin.

Als oberflächenaktive Verbindungen kommen je nach Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen, als auch wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$-$C_{22}$), wie z. B. die Na- oder K-Salze der Oel- oder Stearinsäure, oder von natürlichen Fettsäuregemischen, die z. B. aus Kokosnuss- oder Talgöl gewonnen werden können. Ferner sind auch die Fettsäure-methyllaurinsalze zu erwähnen.

Häufiger werden jedoch sog. synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkalirest mit 8 bis 22 C-Atomen auf, wobei Alkyl auch den Alkylteil von Acylresten einschliesst, z. B. das Na- oder Ca-Salz der Ligninsulfonsäure, des Dodecylschwefelsäureesters oder eines aus natürlichen Fettsäuren hergestellten Fettalkoholsulfatgemisches.

Hierhier gehören auch die Salze der Schwefelsäureester und Sulfonsäuren von Fettalkohol-Ethylenoxyd-Addukten. Die sulfonierten Benzimidazolderivate enthalten, vorzugsweise 2-Sulfonsäuregruppen und einen Fettsäurerest mit 8-22 C-Atomen. Alkylarylsulfonate sind z. B. die Na-, Ca- oder Triethanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z. B. Salze des Phosphorsäureesters eines p-Nonylphenol-(4-14)-Ethylenoxid-Adduktes in Frage.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykolethergruppen und 10 bis 100 Propylenglykolethergruppen enthaltenden Polyethylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykoleinheiten.

Als Beispiele nichtionischer Tenside seien Nonylphenolpolyethoxyethanole, Ricinusölpolyglycolether, Polypropylen-Polyethylenoxydaddukte, Tributylphenoxypolyethoxyethanol, Polyethylenglykol und Octylphenoxypolyethoxyethanol erwähnt.

Ferner kommen auch Fettsäureester von Polyoxyethylensorbitan wie das Polyoxyethylensorbitantrioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z. B. das Stearyltrimethylammoniumchlorid oder das Benzyldi(2-chlorethyl) ethylammoniumbromid.

Die in der Formulierungstechnik gebräuchlichen Tenside sind u. a. in folgenden Publikationen beschrieben :

« Mc Cutcheon's Detergents and Emulsifiers Annual » MC Publishing Corp., Ridgewood New Jersey, 1981

Dr. Helmut Stache « Tensid-Taschenbuch » Carl Hanser Verlag München/Wien 1981

Die agrochemischen Zubereitungen enthalten in der Regel 0,1 bis 99 %, insbesondere 0,1 bis 95 % Wirkstoff der Formel I, 99,9 bis 1 %, insbesondere 99,8 bis 5 % eines festen oder flüssigen Zusatzstoffes und 0 bis 25 %, insbesondere 0,1 bis 25 % eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Derartige agrochemische Mittel sind ein Bestandteil der vorliegenden Erfindung.

Die nachfolgenden Beispiele dienen zur näheren Erläuterung der Erfindung, ohne dieselbe einzuschränken. Temperaturen sind in Celsiusgraden angegeben. Prozente und Teile beziehen sich auf das Gewicht. RT bedeutet Raumtemperatur, h steht für Stunde.

Herstellungsbeispiele :

Beispiel H1 : Herstellung von

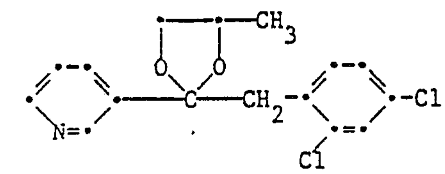

(2.1)

3-[2-(2,4-Dichlorbenzyl)-4-methyl-1,3-dioxolan-2-yl] pyridin

13,3 g 2,4-Dichlorbenzyl-3-pyridyl-keton, 7,6 g Propandiol-1,2 und 13,0 g p-Toluolsulfonsäure werden in 150 ml Xylol 32 h am Wasserabscheider gekocht. Nach dem Abkühlen auf RT wird das Reaktionsgemisch auf 600 ml 2n Natronlauge gegossen und 3 x mit je 200 ml Ether extrahiert. Die vereinigten, organischen Phasen werden mit 250 ml Wasser gewaschen, über Natriumsulfat getrocknet und filtriert. Das Lösungsmittel wird verdampft und das ölige Rohprodukt säulenchromatographisch gereinigt. (Laufmittel Ether). Die Verbindung wird als farbloses, klares Oel erhalten. $n_D^{22.5}$ : 1,5676

Beispiel H2 : Herstellung von

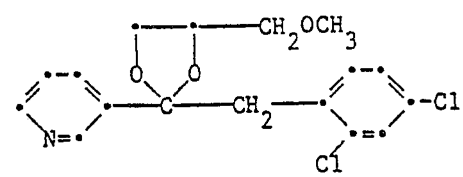

(2.12)

3-[2-(2,4-Dichlorbenzyl)-4-methoxymethyl-1,3-dioxolan-2-yl] pyridin

6,0 g 3-[2-(2,4-Dichlorbenzyl)-4-chlormethyl-1,3-dioxolan-2-yl]-pyridin, 1,8 g Natriummethylat und eine katalytisch wirkende Menge Natriumjodid werden in 50 ml Dimethylsulfoxid 20 h bei einer Innentemperatur von + 120° gerührt. Nach dem Abkühlen auf RT wird das Reaktionsgemisch auf 400 ml Wasser gegossen und 2 x mit je 120 ml Essigester extrahiert. Die organischen Phasen werden vereinigt, mit 150 ml Wasser gewaschen, über Natriumsulfat getrocknet, mit Aktivkohle behandelt, filtriert und das Lösungsmittel verdampft. Der ölige Rückstand wird durch Chromatographie an Kieselgel mit Ether gereinigt. Die Verbindung wird als farbloses Oel erhalten. $n_D^{22.5}$ : 1,4774.

Auf analoge Weise lassen sich auch die nachfolgenden Endprodukte (falls nicht besonders vermerkt Diastereomerengemische mit unterschiedlichen Mischungsverhältnissen) der Formel I herstellen :

(Siehe Tabelle 1 Seite 11 f.)

Tabelle 1 : Verbindungen der Formel

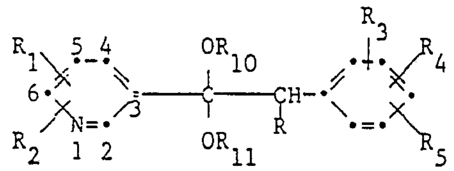

| Verb. Nr. | $R_1$ | $R_2$ | $R_{10}$ | $R_{11}$ | R | $R_3$ | $R_4$ | $R_5$ | Salz |
|---|---|---|---|---|---|---|---|---|---|
| 1.1 | H | H | $CH_3$ | $CH_3$ | H | 2-Cl | 4-Cl | H | - |
| 1.2 | 2-Cl | H | $CH_3$ | $CH_3$ | $CH_3$ | 2-Cl | 4-Cl | H | - |
| 1.3 | H | 6-Cl | $CH_3$ | $CH_3$ | H | 2-Cl | 4-Cl | H | - |
| 1.4 | 2-Cl | H | $CH_3$ | $CH_3$ | H | 2-Cl | 4-Cl | H | - |
| 1.5 | 2-Cl | H | $C_2H_5$ | $C_2H_5$ | $C_3H_7$-n | 2-Cl | 4-Cl | H | $HNO_3$ |
| 1.6 | H | 6-$OCH_3$ | $CH_3$ | $CH_3$ | H | 2-Cl | 4-Cl | H | - |
| 1.7 | 2-Cl | H | $C_2H_5$ | $C_2H_5$ | H | 2-F | H | H | - |
| 1.8 | 2-Cl | H | $CH_3$ | $CH_3$ | H | H | 4-F | H | - |
| 1.9 | H | 6-$CH_3$ | $CH_3$ | $CH_3$ | H | 2-Cl | 4-Cl | H | - |
| 1.10 | H | 6-$CH_3$ | $C_2H_5$ | $C_2H_5$ | H | H | 4-Cl | H | - |
| 1.11 | H | 6-$CH_3$ | $CH_3$ | $CH_3$ | $CH_3$ | H | 4-F | H | - |
| 1.12 | H | 6-$CH_3$ | $CH_3$ | $CH_3$ | $C_6H_5$ | 2-Cl | 4-Cl | 6-Cl | - |
| 1.13 | 2-Cl | H | $CH_3$ | $CH_3$ | H | H | 4-$CH_3$ | H | - |
| 1.14 | H | 6-Cl | $CH_3$ | $CH_3$ | H | H | 4-$CH_3$ | H | - |
| 1.15 | H | 6-$OCH_3$ | $CH_3$ | $CH_3$ | $CH_3$ | H | 3-$CF_3$ | H | HCl |
| 1.16 | H | 6-$CH_3$ | $C_2H_5$ | $C_2H_5$ | H | H | 3-$CF_3$ | H | - |
| 1.17 | H | H | $CH_3$ | $CH_3$ | H | H | 3-$CF_3$ | H | - |
| 1.18 | 2-Cl | H | $CH_3$ | $CH_3$ | $CH_2C_6H_5$ | 2-Cl | 4-Cl | H | - |
| 1.19 | H | H | $CH_3$ | $CH_3$ | 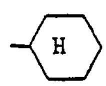 | 2-Cl | 4-Cl | H | - |
| 1.20 | H | H | $CH_3$ | $CH_3$ | H | H | 4-$C_6H_5$ | H | - |
| 1.21 | H | 6-$OCH_3$ | $C_2H_5$ | $C_2H_5$ | H | H | 4-$NO_2$ | H | - |
| 1.22 | H | H | $C_2H_5$ | $C_2H_5$ | H | 2-Cl | 4-Cl | H | - |
| 1.23 | H | H | $CH_3$ | $CH_3$ | $CH_3$ | 2-Cl | 4-Cl | H | - |

Tabelle 2 : Verbindungen der Formel

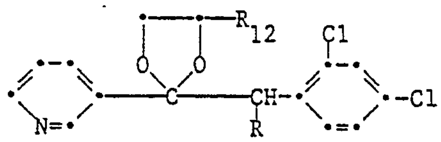

einschliesslich aller isomerer Formen

| Verb. Nr. | $R_{12}$ | R | Salz | Physikalische Konstante |
|---|---|---|---|---|
| 2.1 | $CH_3$ | H | - | $n_D^{22,5}$ : 1,5676 |
| 2.2 | $CH_3$ | $CH_3$ | $HNO_3$ | |
| 2.3 | $CH_3$ | $CH_3$ | HCl | |
| 2.4 | $CH_3$ | $CH_3$ | - | |
| 2.5 | $CH_3$ | $CH_2C_6H_5$ | $(COOH)_2$ | |
| 2.6 | $C_2H_5$ | H | - | $n_D^{24,5}$ : 1,5651 |
| 2.7 | $C_2H_5$ | H | $(COOH)_2$ | |
| 2.8 | $C_2H_5$ | $CH_3$ | - | |
| 2.9 | $C_3H_7$-i | H | - | |
| 2.10 | $C_3H_7$-n | H | | |
| 2.11 | $C_3H_7$-n | $C_2H_5$ | - | |
| 2.12 | $CH_2OCH_3$ | H | - | $n_D^{22,5}$ : 1,4774 |
| 2.13 | $CH_2OCH_3$ | $CH_3$ | - | |
| 2.14 | $CH_2OCH_3$ | $C_6H_5$ | - | |
| 2.15 | $CH_2OC_2H_5$ | H | - | |
| 2.16 | $CH_2OH$ | H | - | $n_D^{26}$ : 1,5651 |
| 2.17 | $CH_2OH$ | H | $CCl_3COOH$ | |
| 2.18 | $CH_2OC_6H_5$ | H | - | |
| 2.19 | $CH_2OCH_2CH=CH_2$ | H | - | |
| 2.20 | $C_3H_7$-n | H | $HNO_3$ | |
| 2.21 | $C_4H_9$-n | H | - | |
| 2.22 | $CH_2OCH_2CH=CH_2$ | $CH_3$ | - | |
| 2.23 | $CH_2OCH_2CH=CH_2$ | 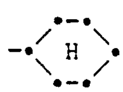 | - | |
| 2.24 | $CH_2OC_6H_5$ | $C_3H_7$-n | - | |
| 2.25 | $CH_2Cl$ | H | - | $n_D^{22,5}$ : 1,5703 |
| 2.26 | $CH_2Cl$ (trans) | H | - | $n_D^{21}$ : 1,5688 |
| 2.27 | $CH_2Cl$ (cis) | H | - | $n_D^{21}$ : 1,5736 |
| 2.28 | $CH_2Cl$ | $CH_3$ | - | $n_D^{22}$ : 1,5656 |
| 2.29 | $CH_2Cl$ | $C_6H_5$ | | |

Tabelle 2 (Fortsetzung)

| Verb. Nr. | $R_{12}$ | R | Salz | Physikalische Konstante |
|---|---|---|---|---|
| 2.30 | $CH_2Cl$ | 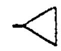 | - | |
| 2.31 | $CH_2Cl$ | $CH_2(C_6H_3Cl_2$-2,4) | - | |
| 2.32 | $CH_2CH_3$ | $C_4H_9$-t | - | |
| 2.33 | $CH_2Cl$ | H | HCl | |
| 2.34 | $CH_2Cl$ | $C_2H_5$ | - | Harz |
| 2.35 | $CH_2Cl$ | $C_3H_7$-n | - | Harz |
| 2.36 | $CH_2Cl$ | $CH_2C_6H_5$ | - | Harz |
| 2.37 | $CH_2Br$ | H | - | Harz |
| 2.38 | $CH_2OH$ | $CH_3$ | - | Harz |
| 2.39 | $CH_2Cl$(trans) | $CH_3$ | - | $n_D^{22}$ : 1,5623 |
| 2.40 | $CH_2Cl$(cis) | $CH_3$ | - | $n_D^{22}$ : 1,5675 |

Tabelle 3 : Verbindungen der Formel

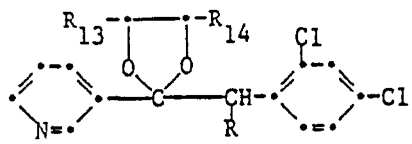

einschliesslich ihrer isomerer Formen

| Verb. Nr. | $R_{13}$ | $R_{14}$ | R | Salz | Physikalische Konstante |
|---|---|---|---|---|---|
| 3.1 | $CH_3$ | $C_2H_5$ | H | - | |
| 3.2 | $CH_3$ | $C_2H_5$ | $CH_3$ | - | |
| 3.3 | $CH_3$ | $C_2H_5$ | H | $HNO_3$ | |
| 3.4 | $CH_3$ | $C_3H_7$-n | H | - | |
| 3.5 | $CH_3$ | $C_3H_7$-n | $CH_3$ | - | |
| 3.6 | $CH_3$ | $C_2H_5$ | $C_2H_5$ | HCl | |
| 3.7 | $CH_3$ | $CH_3$ | H | - | Harz |
| 3.8 | $CH_3$ | $CH_3$ | $CH_3$ | - | Harz |
| 3.9 | $C_2H_5$ | $C_3H_7$-n | H | - | |
| 3.10 | $C_2H_5$ | $C_3H_7$-i | H | - | |
| 3.11 | $C_2H_5$ | $C_2H_5$ | H | - | |
| 3.12 | $CH_3$ | $C_2H_5$ | $C_6H_5$ | - | |
| 3.13 | $CH_3$ | $C_2H_5$ | $CH_2C_6H_5$ | - | |
| 3.14 | $CH_3$ | $C_2H_5$ | Cyclohexyl | - | |
| 3.15 | $CH_3$ | $C_3H_7$-i | $C_6H_5$ | $(COOH)_2$ | |
| 3.16 | $CH_3$ | $C_2H_5$ | $C_3H_7$-n | - | |

Tabelle 3 (Fortsetzung)

| Verb. Nr. | $R_{13}$ | $R_{14}$ | R | Salz | Physikalische Konstante |
|---|---|---|---|---|---|
| 3.17 | $CH_3$ | $CH_3$ | 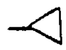 | - | |
| 3.18 | $CH_3$ | $CH_3$ | $C_3H_7$-i | - | |
| 3.19 | $-(CH_2)_4-$ | | H | - | |
| 3.20 | $-(CH_2)_4-$ | | $CH_3$ | - | |
| 3.21 | $-(CH_2)_4-$ | | $C_6H_5$ | - | |
| 3.22 | $-(CH_2)_4-$ | | H | HCl | |

Tabelle 4 : Verbindungen der Formel

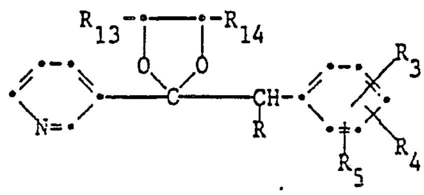

einschliesslich ihrer isomerer Formen

| Verb. Nr. | $R_{13}$ | $R_{14}$ | R | $R_3$ | $R_4$ | $R_5$ | Salz |
|---|---|---|---|---|---|---|---|
| 4.1 | H | $CH_3$ | H | 3-Cl | 4-Cl | H | - |
| 4.2 | H | $CH_3$ | H | 3-Cl | 4-Cl | H | HCl |
| 4.3 | $CH_3$ | $CH_3$ | H | 3-$NO_2$ | H | H | - |
| 4.4 | $CH_3$ | $C_2H_5$ | H | 3-$CF_3$ | H | H | - |
| 4.5 | H | $CH_3$ | $CH_3$ | 2-Cl | H | H | - |
| 4.6 | H | $CH_2Cl$ | H | 3-F | H | H | - |
| 4.7 | H | $CH_2Cl$ | $CH_3$ | 2-Br | 4-Br | H | - |
| 4.8 | H | $CH_2OCH_3$ | H | 2-F | H | H | - |
| 4.9 | H | $CH_2OCH_3$ | H | H | 3-$CF_3$ | H | - |
| 4.10 | H | $CH_2OCH_3$ | H | H | 4-F | H | - |
| 4.11 | H | $CH_2OCH_3$ | H | H | 4-Cl | H | - |
| 4.12 | H | $CH_2Cl$ | H | H | 4-$C_6H_5$ | H | - |
| 4.13 | H | $CH_2Cl$ | H | H | H | H | - |
| 4.14 | H | $CH_2OH$ | $CH_3$ | H | 4-Cl | H | - |
| 4.15 | H | $CH_2OH$ | H | H | 4-F | H | - |
| 4.16 | H | $CH_2OH$ | $CH_3$ | H | 4-$CH_3$ | H | - |
| 4.17 | H | $CH_2OH$ | $C_6H_5$ | 2-Cl | 4-Cl | 6-Cl | $(COOH)_2$ |
| 4.18 | H | $CH_2OC_6H_5$ | H | H | 4-F | H | - |
| 4.19 | H | $CH_2OCH_2CH{=}CH_2$ | $CH_3$ | H | 4-Cl | H | - |

Tabelle 4 (Fortsetzung)

| Verb. Nr. | $R_{13}$ | $R_{14}$ | R | $R_3$ | $R_4$ | $R_5$ | Salz |
|---|---|---|---|---|---|---|---|
| 4.20 | H | $CH_2Cl$ | $C_6H_5$ | H | $4-CH_3$ | H | - |
| 4.21 | H | $CH_2Cl$ | $CH_2C_6H_5$ | H | 4-F | H | - |
| 4.22 | H | $CH_2Cl$ | H | $3-CF_3$ | H | H | - |
| 4.23 | $-(CH_2)_4-$ | | H | H | 4-Cl | H | - |
| 4.24 | $-(CH_2)_4-$ | | $CH_3$ | H | 4-Cl | H | - |
| 4.25 | $-(CH_2)_4-$ | | $C_3H_7-n$ | H | 4-F | H | - |
| 4.26 | $-(CH_2)_4-$ | | $C_6H_5$ | H | $3-CF_3$ | H | - |

Tabelle 5 : Verbindungen der Formel

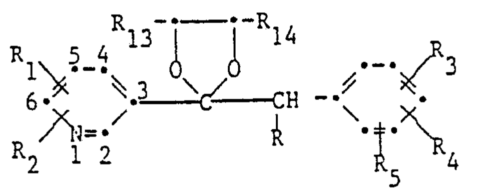

mit $R_5 = H$, $R_2 = H$

| Verb. Nr. | $R_1$ | $R_{13}$ | $R_{14}$ | $R_3$ | $R_4$ | R | Salz | Physik. Konstante |
|---|---|---|---|---|---|---|---|---|
| 5.1 | 2-Cl | H | $CH_3$ | 2-Cl | 4-Cl | H. | - | |
| 5.2 | 2-Cl | $CH_3$ | $CH_3$ | H | 4-Cl | H | - | |
| 5.3 | 2-Cl | $CH_3$ | $C_2H_5$ | H | 4-F | $CH_3$ | HCl | |
| 5.4 | 2-Cl | H | $CH_2OCH_3$ | H | $3-CF_3$ | H | - | |
| 5.5 | 6-Cl | H | $CH_2OCH_3$ | 2-Cl | 4-Cl | H | - | |
| 5.6 | 6-Cl | $CH_3$ | $CH_3$ | H | 4-Cl | H | - | |
| 5.7 | 6-Cl | $CH_3$ | $C_2H_5$ | H | $3-CF_3$ | H | - | |
| 5.8 | 6-Cl | H | $C_3H_7-n$ | H | $4-CH_3$ | H | $HNO_3$ | |
| 5.9 | $6-CH_3$ | H | $CH_2OCH_3$ | 2-Cl | 4-Cl | H | - | |
| 5.10 | 6-Cl | $CH_3$ | $CH_3$ | 2-Cl | 4-Cl | H | | Harz |
| 5.11 | $6-CH_3$ | H | $CH_2C_6H_5$ | H | 4-Cl | H | - | |
| 5.12 | $6-OCH_3$ | H | $CH_2OH$ | H | 4-F | H | - | |
| 5.13 | $6-OCH_3$ | $CH_3$ | $CH_3$ | 2-Cl | 4-Cl | H | - | |
| 5.14 | $6-OCH_3$ | $CH_3$ | $C_2H_5$ | 2-Cl | 4-Cl | $CH_3$ | - | |
| 5.15 | 2-Cl | $-(CH_2)_4-$ | | 2-Cl | 4-Cl | H | - | |
| 5.16 | 6-Cl | $-(CH_2)_4-$ | | H | 4-F | H | - | |
| 5.17 | $6-CH_3$ | $-(CH_2)_4-$ | | H | 4-Cl | H | - | |
| 5.18 | $6-OCH_3$ | $-(CH_2)_4-$ | | H | $3-CF_3$ | H | - | |
| 5.19 | 2-Cl | H | $CH_2Cl$ | 2-Cl | 4-Cl | H | $HNO_3$ | |
| 5.20 | 6-Cl | H | $CH_2Cl$ | 2-Cl | 4-Cl | H | - | |

Tabelle 5 (Fortsetzung)

| Verb. Nr. | $R_1$ | $R_{13}$ | $R_{14}$ | $R_3$ | $R_4$ | R | Salz | Physik. Konstante |
|---|---|---|---|---|---|---|---|---|
| 5.21 | 6-$OCH_3$ | H | $CH_2Cl$ | 2-Br | 4-Br | H | - | |
| 5.22 | 6-$CH_3$ | H | $CH_2Cl$ | H | 4-Cl | H | - | |
| 5.23 | 2-Cl | H | $CH_2Cl$ | 2-Cl | 4-Cl | $C_2H_5$ | - | |
| 5.24 | 6-Cl | H | $CH_2Cl$ | 2-Cl | 4-Cl | $CH_2[C_6H_3(Cl_2)_{2,4}]$ | - | |
| 5.25 | 2-Cl | H | $CH_2Cl$ | 2-Cl | 4-Cl | $C_6H_5$ | | |
| 5.26 | 6-$OCH_3$ | H | $C_2H_5$ | 2-Cl | 4-Cl | Cyclohexyl | - | |
| 5.27 | 2-Cl | $CH_3$ | H | 2-Cl | 4-Cl | H | - | Harz |
| 5.28 | 2-Cl | $CH_2Cl$ | H | 2-Cl | 4-Cl | H | - | Harz |
| 5.29 | 2-Cl | $CH_2OH$ | H | 2-Cl | 4-Cl | H | - | Harz |
| 5.30 | 2-Cl | $CH_2Cl$ | H | 2-Cl | 4-Cl | $CH_3$ | - | Harz |
| 5.31 | 6-Cl | $CH_3$ | H | 2-Cl | 4-Cl | H | - | Harz |
| 5.32 | 6-Cl | $CH_2OH$ | H | 2-Cl | 4-Cl | H | - | Harz |
| 5.33 | 6-Cl | $CH_2Cl$ | H | 2-Cl | 4-Cl | H | - | Harz |
| 5.34 | 6-Cl | $CH_2Cl$ | H | 2-Cl | 4-Cl | $CH_3$ | - | Harz |
| 5.35 | 6-Cl | $CH_2Cl$ | H | 2-Cl | 4-Cl | $CH_2C_6H_5$ | - | Harz |

Tabelle 6 : Verbindungen der Formel

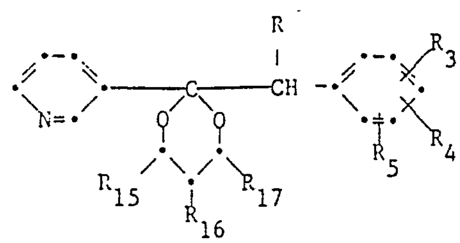

mit $R_5 = H$

| Verb. Nr. | $R_{15}$ | $R_{16}$ | $R_{17}$ | $R_3$ | $R_4$ | R | Salz | Physik. Konstante |
|---|---|---|---|---|---|---|---|---|
| 6.1 | $CH_3$ | H | H | 2-Cl | 4-Cl | H | - | |
| 6.2 | H | $CH_3$ | H | 2-Cl | 4-Cl | H | HCl | |
| 6.3 | H | $CH_3$ | $CH_3$ | 2-Cl | 4-Cl | H | - | |
| 6.4 | $CH_3$ | $CH_3$ | $CH_3$ | 2-Cl | 4-Cl | H | - | |
| 6.5 | H | H | H | 2-Cl | 4-Cl | H | - | Smp. 82-84° |
| 6.6 | H | $C_2H_5$ | H | H | 4-F | H | - | |
| 6.7 | $CH_3$ | $CH_3$ | H | H | 3-$CF_3$ | H | - | |
| 6.8 | $C_2H_5$ | H | H | 2-Cl | 4-Cl | H | - | |
| 6.9 | $C_2H_5$ | H | H | H | 4-$CH_3$ | H | - | |
| 6.10 | $C_3H_7$-n | H | $C_3H_7$-n | H | 4-F | H | - | |
| 6.11 | $C_2H_5$ | H | $C_2H_5$ | H | 4-Cl | H | - | |

Tabelle 6 (Fortsetzung)

| Verb. Nr. | $R_{15}$ | $R_{16}$ | $R_{17}$ | $R_3$ | $R_4$ | R | Salz | Physik. Konstante |
|---|---|---|---|---|---|---|---|---|
| 6.12 | H | H | $C_4H_9$-n | 2-Cl | 4-Cl | H | $HNO_3$ | |
| 6.13 | H | $C_3H_7$-i | $CH_3$ | H | 4-$C_6H_5$ | H | - | |
| 6.14 | $CH_3$ | H | H | 2-Cl | 4-Cl | -$CH_3$ | - | |
| 6.15 | $CH_3$ | H | H | 2-Cl | 4-Cl | $CH_2[C_6H_3Cl_2(2,4)]$ | - | |
| 6.16 | H | H | $CH_3$ | 2-Cl | 4-Cl | Cyclohexyl | - | |
| 6.17 | $C_2H_5$ | H | H | 2-Cl | 4-Cl | $C_6H_5$ | - | |
| 6.18 | $C_2H_5$ | H | H | 2-Cl | 4-Cl | $C_6H_{12}$-n | - | |
| 6.19 | H | H | H | 2-Cl | 4-Cl | $CH_3$ | - | Harz |
| 6.20 | H | H | H | 2-Cl | 4-Cl | $C_3H_7$-n | - | Harz |
| 6.21 | H | H | H | 2-Cl | 4-Cl | H | $HNO_3$ | Smp. 138-140° |

Tabelle 7 : Verbindungen der Formel

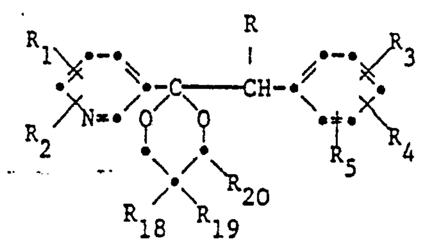

$R_2 + R_5 = H$

| Verb. Nr. | $R_1$ | $R_{18}$ | $R_{19}$ | $R_{20}$ | $R_3$ | $R_4$ | R | Phys. Konst. |
|---|---|---|---|---|---|---|---|---|
| 7.1 | 2-Cl | H | H | $CH_3$ | 2-Cl | 4-Cl | H | |
| 7.2 | H | $CH_3$ | $CH_3$ | H | 2-Cl | 4-Cl | H | |
| 7.3 | H | $CH_3$ | $C_2H_5$ | H | 2-Cl | 4-Cl | H | |
| 7.4 | H | $CH_3$ | $CH_3$ | $CH_3$ | H | 4-$C_6H_5$ | $CH_3$ | |
| 7.5 | 6-Cl | H | H | $CH_3$ | 2-Cl | 4-Cl | $C_3H_7$-i | |
| 7.6 | 2-$OCH_3$ | $CH_3$ | $CH_3$ | H | H | H | H | |
| 7.7 | H | $C_2H_5$ | $C_2H_5$ | H | H | 3-$CF_3$ | $C_6H_5$ | |
| 7.8 | 6-Cl | H | H | $CH_3$ | H | 3-$CF_3$ | H | |
| 7.9 | 2-$CH_3$ | $CH_3$ | $CH_3$ | H | H | 3-$NO_2$ | $CH_2C_6H_5$ | |
| 7.10 | H | $CH_3$ | $CH_3$ | H | 2-F | H | H | |
| 7.11 | 2-Cl | H | H | H | 2-Cl | 4-Cl | H | Harz |
| 7.12 | 6-Cl | H | H | $CH_3$ | 2-Cl | 4-Cl | H | Harz |
| 7.13 | 6-Cl | H | H | H | 2-Cl | 4-Cl | H | Harz |

Formulierungsbeispiele für flüssige Wirkstoffe der Formel I (% : Gewichtsprozent)

| F1. Emulsions-Konzentrate | a) | b) | c) |
|---|---|---|---|
| Wirkstoff aus den Tabellen | 25% | 40% | 50% |
| Ca-Dodecylbenzolsulfonat | 5% | 8% | 6% |
| Ricinusöl-polyethylenglykolether (36 Mol Ethylenoxid) | 5% | - | - |
| Tributylphenoyl-polyethylenglykol-ehter (30 Mol Ethylenoxid) | - | 12% | 4% |
| Cyclohexanon | - | 15% | 20% |
| Xylolgemisch | 65% | 25% | 20% |

Aus solchen Konzentraten können durch Verdünnen mit Wasser Emulsionen jeder gewünschten Konzentration hergestellt werden.

| F2. Lösungen | a) | b) | c) | d) |
|---|---|---|---|---|
| Wirkstoff aus den Tabellen | 80% | 10% | 5% | 95% |
| Ethylenglykol-monomethyl-ether | 20% | - | - | - |
| Polyethylenglykol MG 400 | - | 70% | - | - |
| N-Methyl-2-pyrrolidon | - | 20% | - | - |
| Epoxydiertes Kokosnussöl | - | - | 1% | 5% |
| Benzin (Siedegrenzen 160-190°C) | - | - | 94% | - |

(MG = Molekulargewicht)

Die Lösungen sind zur Anwendung in Form kleinster Tropfen geeignet.

| F3. Granulate | a) | b) |
|---|---|---|
| Wirkstoff aus den Tabellen | 5% | 10% |
| Kaolin | 94% | - |
| Hochdisperse Kieselsäure | 1% | - |
| Attapulgit | - | 90% |

Der Wirkstoff wird in Methylenchlorid gelöst, auf den Träger aufgesprüht und das Lösungsmittel anschliessend im Vakuum abgedampft.

| F4. Stäubemittel | a) | b) |
|---|---|---|
| Wirkstoff aus den Tabellen | 2% | 5% |
| Hochdisperse Kieselsäure | 1% | 5% |
| Talkum | 97% | - |
| Kaolin | - | 90% |

Durch inniges Vermischen auf Trägerstoffe mit dem Wirkstoff erhält man gebrauchsfertige Stäubemittel.

Formulierungsbeispiele für feste Wirkstoffe der Formel I (% = Gewichtsprozent)

| F5. Spritzpulver | a) | b) | c) |
|---|---|---|---|
| Wirkstoff aus den Tabellen | 25% | 50% | 75% |
| Na-Ligninsulfonat | 5% | 5% | - |
| Na-Laurylsulfat | 3% | - | 5% |
| Na-Diisobutylnaphthalinsulfonat | - | 6% | 10% |
| Octylphenolpolyethylenglykolether (7-8 Mol Ethylenoxid) | - | 2% | - |
| Hochdisperse Kieselsäure | 5% | 10% | 10% |
| Kaolin | 62% | 27% | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

F6. Emulsions-Konzentrat

| | |
|---|---|
| Wirkstoff aus den Tabellen | 10 % |
| Octylphenolpolyethylenglykolether (4-5 Mol Ethylenoxid) | 3 % |
| Ca-Dodecylbenzolsulfonat | 3 % |
| Ricinusölpolyglykolether (35 Mol Ethylenoxid) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| F7. Stäubemittel | a) | b) |
|---|---|---|
| Wirkstoff aus den Tabellen | 5% | 8% |
| Talkum | 95% | - |
| Kaolin | - | 92% |

Man erhält anwendungsfertige Stäubemittel, indem der Wirkstoff mit den Träger vermischt und auf einer geeigneten Mühle vermahlen wird.

F8. Extruder Granulat

| | |
|---|---|
| Wirkstoff aus den Tabellen | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

F9. Umhüllungs-Granulat

| | |
|---|---|
| Wirkstoff aus den Tabellen | 3 % |
| Polyethylenglykol (MG 200) | 3 % |
| Kaolin (MG : Molekulargewicht) | 94 % |

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

F10. Suspensions-Konzentrat

| | |
|---|---|
| Wirkstoff aus den Tabellen | 40 % |
| Ethylenglykol | 10 % |
| Nonylphenolpolyethylenglykolether (15 Mol Ethylenoxid) | 6 % |

| | |
|---|---|
| N-Ligninsulfonat | 10 % |
| Carboxymethylcellulose | 1 % |
| 37 %ige wässrige Formaldehyd-Lösung | 0,2 % |
| Silikonöl in Form einer 75 %igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

## Biologische Beispiele

### Beispiel B1 : Wirkung gegen Puccinia graminis auf Weizen

a) Residual-protektive Wirkung

Weizenpflanzen wurden 6 Tage nach der Aussaat mit einer aus Spritzpulver des Wirkstoffes hergestellten Spritzbrühe (0,06 % Aktivsubstanz) besprüht. Nach 24 Stunden wurden die behandelten Pflanzen mit einer Uredosporensuspension des Pilzes infiziert. Nach einer Inkubation während 48 Stunden bei 95-100 % relativer Luftfeuchtigkeit und ca. 20 °C wurden die infizierten Pflanzen in einem Gewächshaus bei ca. 22 °C aufgestellt. Die Beurteilung der Rostpustelnentwicklung erfolgte 12 Tage nach der Infektion.

b) Systemische Wirkung

Zu Weizenpflanzen wurde 5 Tage nach der Aussaat eine aus Spritzpulver des Wirkstoffes hergestellte Spritzbrühe gegossen (0,006 % Aktivsubstanz bezogen auf das Bodenvolumen). Nach 48 Stunden wurden die behandelten Pflanzen mit einer Uredosporensuspension des Pilzes infiziert. Nach einer Inkubation während 48 Stunden bei 95-100 % relativer Luftfeuchtigkeit und ca. 20 °C wurden die infizierten Pflanzen in einem Gewächshaus bei ca. 22 °C aufgestellt. Die Beurteilung der Rostpustelnentwicklung erfolgte 12 Tage nach der Infektion.

Verbindungen aus den Tabellen zeigten gegen Puccinia-Pilze eine gute Wirkung. Unbehandelte aber infizierte Kontrollpflanzen zeigten einen Puccinia-Befall von 100 %. Unter anderen hemmten die Verbindungen Nr. 2.1, 2.25 bis 2.27 sowie 2.38 bis 2.40 den Puccinia-Befall auf 0 bis 5 %.

### Beispiel B2 : Wirkung gegen Cercospora arachidicola auf Erdnusspflanzen

a) Residual-protektive Wirkung

10-15 cm hohe Erdnusspflanzen wurden mit einer aus Spritzpulver der Wirksubstanz hergestellten Spritzbrühe (0,02 % Aktivsubstanz) besprüht und 48 Stunden später mit einer Konidiensuspension des Pilzes infiziert. Die infizierten Pflanzen wurden während 72 Stunden bei ca. 21 °C und hoher Luftfeuchtigkeit inkubiert und anschliessend bis zum Auftreten der typischen Blattflecken in einem Gewächshaus aufgestellt. Die Beurteilung der fungiziden Wirkung erfolgte 12 Tage nach der Infektion basierend auf Anzahl und Grösse der auftretenden Flecken.

b) Systemische Wirkung

Zu 10-15 cm hohen Erdnusspflanzen wurde eine aus Spritzpulver des Wirkstoffes hergestellte Spritzbrühe gegossen (0,06 % Aktivsubstanz bezogen auf das Erdvolumen). Nach 48 Stunden wurden die behandelten Pflanzen mit einer Konidiensuspension des Pilzes infiziert und 72 Stunden bei ca. 21 °C und hoher Luftfeuchtigkeit inkubiert. Anschliessend wurden die Pflanzen im Gewächshaus aufgestellt und nach 11 Tagen der Pilzbefall beurteilt.

Im Vergleich zu unbehandelten, aber infizierten Kontrollpflanzen (Anzahl und Grösse der Flecken : 100 %), zeigten Erdnusspflanzen, die mit Wirkstoffen aus den Tabellen behandelt wurden, einen stark reduzierten Cercospora-Befall. So verhinderten die Verbindungen Nr. 2.1, 2.6, 2.16, 2.25 bis 2.28, 2.34, 2.38 bis 2.40, 3.7, 3.8 und 6.5 in obigen Versuchen das Auftreten von Flecken fast vollständig (0-10 %).

### Beispiel B3 : Wirkung gegen Erysiphae graminis auf Gerste

a) Residual-protektive Wirkung

Ca. 8 cm hohe Gerstenpflanzen wurden mit einer aus Spritzpulver des Wirkstoffes hergestellten Spritzbrühe (0,02 % Aktivsubstanz) besprüht. Nach 3-4 Stunden wurden die behandelten Pflanzen mit Konidien des Pilzes bestäubt. Die infizierten Gerstenpflanzen wurden in einem Gewächshaus bei ca. 22 °C aufgestellt und der Pilzbefall nach 10 Tagen beurteilt.

b) Systemische Wirkung

Zu ca. 8 cm hohen Gerstenpflanzen wurde eine aus Spritzpulver des Wirkstoffes hergestellte Spritzbrühe gegossen (0,006 % Aktivsubstanz bezogen auf das Erdvolumen). Es wurde dabei darauf geachtet, dass die Spritzbrühe nicht mit den oberirdischen Pflanzenteilen in Berührung kam. Nach 48 Stunden wurden die behandelten Pflanzen mit Konidien des Pilzes bestäubt. Die infizierten Gerstenpflanzen wurden in einem Gewächshaus bei ca. 22 °C aufgestellt und der Pilzbefall nach 10 Tagen beurteilt.

Verbindungen der Formel I zeigten eine gute Wirkung gegen Erysiphe-Pilze. Unbehandelte, aber infizierte Kontrollpflanzen zeigten einen Erysiphe-Befall von 100 %. Unter anderen Verbindungen aus den Tabellen hemmten die Verbindungen Nr. 2.1, 2.6, 2.12, 2.16, 2.25 bis 2.28, 2.34, 2.35, 2.37 bis 2.40, 3.7, 3.8, 5.10, 5.28 bis 5.30, 5.32 bis 5.34, 6.5, 6.19 und 6.21 den Pilzbefall bei Gerste auf 0 bis 5 %.

Beispiel B4 : Residual-protektive Wirkung gegen Venturia inaequalis auf Apfeltrieben

Apfelstecklinge mit 10-20 cm langen Frischtrieben wurden mit einer aus Spritzpulver des Wirkstoffes hergestellten Spritzbrühe (0,06 % Aktivsubstanz) besprüht. Nach 24 Stunden wurden die behandelten Pflanzen mit einer Konidiensuspension des Pilzes infiziert. Die Pflanzen wurden dann während 5 Tagen bei 90-100 % relativer Luftfeuchtigkeit inkubiert und während 10 weiteren Tagen in einem Gewächshaus bei 20-24 °C aufgestellt. Der Schorfbefall wurde 15 Tage nach der Infektion beurteilt. Verbindungen aus den Tabellen hemmten den Krankheitsbefall auf weniger als 10 %. Unbehandelte aber infizierte Triebe zeigten einen 100 %igen Venturia-Befall.

Beispiel B5 : Wirkung gegen Botrytis cinerea auf Bohnen Residual protektive Wirkung

Ca. 10 cm hohe Bohnen-Pflanzen wurden mit einer aus Spritzpulver des Wirkstoffes hergestellten Spritzbrühe (0,02 % Aktivsubstanz) besprüht. Nach 48 Stunden werden die behandelten Pflanzen mit einer Konidiensuspension des Pilzes infizert. Nach einer Inkubation der infizierten Pflanzen während 3 Tagen bei 95-100 % relativer Luftfeuchtigkeit und 21 °C erfolgte die Beurteilung des Pilzbefalls. Die Verbindungen aus den Tabellen hemmten in vielen Fällen die Pilzinfektion sehr stark, insbesondere die Verbindungen Nr. 2.1, 2.6, 2.12, 2.16, 2.25, 2.26, 2.27, 2.28, 2.34, 2.38 bis 2.40, 3.8 und 6.5.

**Patentansprüche** (für die Vertragsstaaten : BE, DE, FR, IT, LU, SE, CH, LI)

1. Verbindungen der allgemeinen Formel I

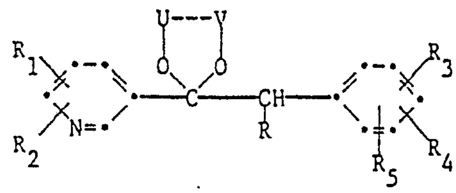

worin

R für Wasserstoff, $C_1$-$C_6$-Alkyl, $C_3$-$C_6$-Cycloalkyl, Phenyl, Benzyl, durch $C_1$-$C_4$-Alkyl, Halogen, $C_1$-$C_4$-Alkoxy, $NO_2$ und/oder $CF_3$ substituiertes Phenyl oder durch $C_1$-$C_4$-Alkyl, Halogen, $C_1$-$C_4$-Alkoxy, $NO_2$ und/oder $CF_3$ substituiertes Benzyl steht :

$R_1$ und $R_2$ unabhängig voneinander für Wasserstoff, $C_1$-$C_4$-Alkyl, Halogen oder $C_1$-$C_4$-Alkoxy stehen ;

$R_3$, $R_4$ und $R_5$ unabhängig voneinander Wasserstoff, Halogen, $CF_3$, $NO_2$, $C_1$-$C_4$-Alkyl, $C_1$-$C_4$-Alkoxy, Phenyl oder durch $C_1$-$C_4$-Alkyl, $C_1$-$C_4$-Alkoxy, Halogen, $NO_2$ und/oder $CF_3$ substituiertes Phenyl bedeuten, U und V unabhängig voneinander für $C_1$-$C_6$-Alkyl oder durch Halogen oder $C_1$-$C_4$-Alkoxy substituiertes Alkyl stehen oder zusammen eine der folgenden Alkylenbrücken

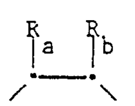 , 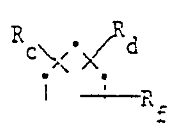 oder 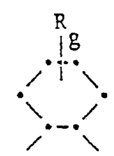 bilden, wobei

$R_a$ für Wasserstoff, $C_1$-$C_{12}$-Alkyl, ein- oder mehrfach durch Halogen substituiertes $C_1$-$C_{12}$-Alkyl, Phenyl, ein- oder mehrfach durch Halogen und/oder $C_1$-$C_4$-Alkyl substituiertes Phenyl oder für die Gruppe $—CH_2—Z—R_h$ steht ;

$R_b$ für $C_1$-$C_{12}$-Alkyl, ein- oder mehrfach durch Halogen substituiertes $C_1$-$C_{12}$-Alkyl, Phenyl, ein- oder

mehrfach durch Halogen und/oder $C_1$-$C_4$-Alkyl substituiertes Phenyl oder für die Gruppe —$CH_2$—Z—$R_h$ steht ;
wobei Z Sauerstoff oder Schwefel bedeutet und

$R_h$ für Wasserstoff, $C_1$-$C_8$-Alkyl, ein durch $C_1$-$C_4$-Alkoxy substituiertes $C_1$-$C_8$-Alkyl, $C_3$-$C_4$-Alkenyl, 2-Propinyl, 3-Halogen-2-propinyl, Phenyl, ein durch Halogen, $C_1$-$C_4$-Alkyl, $C_1$-$C_4$-Alkoxy, $NO_2$ und/oder $CF_3$ substituiertes Phenyl, Benzyl oder durch Halogen, $C_1$-$C_4$-Alkyl, $C_1$-$C_4$-Alkoxy, $NO_2$ und/oder $CF_3$ substituiertes Benzyl steht ;

$R_c$, $R_d$ und $R_f$ unabhängig voneinander Wasserstoff oder $C_1$-$C_4$-Alkyl bedeuten ; und

$R_g$ für Wasserstoff oder $C_1$-$C_4$-Alkyl steht ; unter Einschluss ihrer Säureadditionssalze.

2. Verbindungen der Formel I nach Anspruch 1, worin R für Wasserstoff oder Methyl steht ; $R_1$ und $R_2$ unabhängig voneinander für Wasserstoff, Methyl, Ethyl, Fluor, Chlor, Brom oder Methoxy stehen ; $R_3$, $R_4$ und $R_5$ unabhängig voneinander für Wasserstoff, Fluor, Chlor, Brom, $CF_3$, $NO_2$, Methyl oder Methoxy stehen ; U und V unabhängig voneinander $C_1$-$C_3$-Alkyl bedeuten oder zusammen folgende Alkylenbrücke

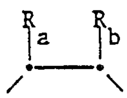

bilden, wobei $R_a$ für Wasserstoff, $C_1$-$C_4$-Alkyl, Phenyl, ein- bis dreifach durch Fluor, Chlor, Brom und/oder Methyl substituiertes Phenyl oder die Gruppe —$CH_2$—Z—$R_h$ steht ; $R_b$ für $C_1$-$C_4$-Alkyl, ein- bis dreifach durch Fluor, Chlor und/oder Brom substituiertes $C_1$-$C_4$-Alkyl, Phenyl, ein- bis dreifach durch Fluor, Chlor, Brom und/oder Methyl substituiertes Phenyl oder die Gruppe —$CH_2$—Z—$R_h$ steht ; wobei Z für Sauerstoff oder Schwefel steht ; $R_h$ Wasserstoff, $C_1$-$C_6$-Alkyl, ein durch $C_1$-$C_4$-Alkoxy substituiertes $C_1$-$C_3$-Alkyl, $C_3$-$C_4$-Alkenyl, 2-Propinyl, 3-Halogen-2-propinyl, Phenyl, ein durch Fluor, Chlor, Brom, Methyl, Methoxy, Nitro und/oder $CF_3$ substituiertes Phenyl, Benzyl oder ein durch Fluor, Chlor, Brom, Methyl, Methoxy, $NO_2$ und/oder $CF_3$ substituiertes Benzyl bedeutet ; unter Einschluss ihrer Säureadditionssalze.

3. Verbindungen der Formel I nach Anspruch 2, worin R für Wasserstoff steht ; $R_1$ und $R_2$ unabhängig voneinander für Wasserstoff, Methyl, Fluor, Chlor, Brom oder Methoxy stehen ; $R_3$, $R_4$ und $R_5$ unabhängig voneinander für Wasserstoff, Fluor, Chlor, Brom, $CF_3$, $NO_2$, Methyl oder Methoxy stehen ; U und V zusammen folgende Alkylenbrücke

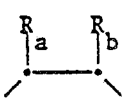

bilden, wobei $R_a$ für Wasserstoff oder Methyl steht ; $R_b$ für $C_1$-$C_4$-Alkyl, durch Fluor, Chlor oder Brom substituiertes $C_1$-$C_4$-Alkyl, Phenyl, ein- bis dreifach durch Fluor, Chlor, Brom und/oder Methyl substituiertes Phenyl oder die Gruppe —$CH_2$—Z—$R_h$ steht ; wobei Z für Sauerstoff oder Schwefel steht ; $R_h$ Wasserstoff, $C_1$-$C_4$-Alkyl, ein durch $C_1$-$C_4$-Alkoxy substituiertes $C_1$-$C_2$-Alkyl, $C_3$-$C_4$-Alkenyl, 2-Propinyl, 3-Halogen-2-propinyl, Phenyl, ein durch Fluor, Chlor, Brom, Methyl, Methoxy, Nitro und/oder $CF_3$ substituiertes Phenyl, Benzyl oder ein durch Fluor, Chlor, Brom, Methyl, Methoxy, Nitro und/oder $CF_3$ substituiertes Benzyl bedeutet.

4. Verbindungen der Formel I nach Anspruch 1, worin R für Wasserstoff oder Methyl steht ; $R_1$ und $R_2$ unabhängig voneinander für Wasserstoff, Methyl, Ethyl, Fluor, Chlor, Brom oder Methoxy stehen ; $R_3$, $R_4$ und $R_5$ unabhängig voneinander für Wasserstoff, Fluor, Chlor, Brom, $CF_3$, $NO_2$, Methyl oder Methoxy stehen ; U und V unabhängig voneinander $C_1$-$C_3$-Alkyl bedeuten oder zusammen folgende Alkylenbrücke

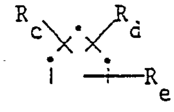

bilden, wobei $R_c$, $R_d$ und $R_e$ unabhängig voneinander für Wasserstoff oder $C_1$-$C_4$-Alkyl stehen und die Summe der Kohlenstoffatome in $R_c$, $R_d$ und $R_e$ die Zahl 6 nicht übersteigt ; unter Einschluss ihrer Säureadditionssalze.

5. Verbindungen der Formel I nach Anspruch 4, worin R für Wasserstoff steht ; $R_1$ und $R_2$ unabhängig voneinander für Wasserstoff, Methyl, Fluor, Chlor, Brom oder Methoxy stehen ; $R_3$, $R_4$ und $R_5$ unabhängig voneinander für Wasserstoff, Fluor, Chlor, Brom, $CF_3$, $NO_2$, Methyl oder Methoxy stehen ; U und V zusammen folgende Alkylenbrücke

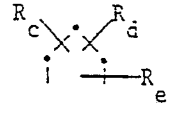

bilden, wobei $R_c$ Wasserstoff bedeutet und $R_d$ und $R_e$ unabhängig voneinander für Wasserstoff, Methyl oder Ethyl stehen.

6. Verbindungen der Formel I nach Anspruch 1, worin R für Wasserstoff oder Methyl steht ; $R_1$ und $R_2$ unabhängig voneinander für Wasserstoff, Methyl, Ethyl, Fluor, Chlor, Brom oder Methoxy stehen ; $R_3$, $R_4$ und $R_5$ unabhängig voneinander für Wasserstoff, Fluor, Chlor, Brom, $CF_3$, $NO_2$, Methyl oder Methoxy stehen ; U und V unabhängig voneinander $C_1$-$C_3$-Alkyl bedeuten oder zusammen folgende Alkylenbrücke

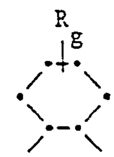

bilden, wobei $R_g$ für Wasserstoff oder $C_1$-$C_4$-Alkyl steht ; unter Einschluss ihrer Säureadditionssalze.

7. Verbindungen der Formel I nach Anspruch 6, worin R für Wasserstoff steht ; $R_1$ und $R_2$ unabhängig voneinander für Wasserstoff, Methyl, Fluor, Chlor, Brom oder Methoxy stehen ; $R_3$, $R_4$ und $R_5$ unabhängig voneinander für Wasserstoff, Fluor, Chlor, Brom, $CF_3$, $NO_2$, Methyl oder Methoxy stehen ; U und V zusammen folgende Alkylenbrücke

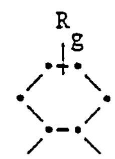

bilden, wobei $R_g$ für Wasserstoff, Methyl oder Ethyl steht.

8. Eine Verbindung der Formel I nach Anspruch 1 als Gemisch ihrer Isomeren oder in optisch reiner Form ausgewählt aus der Reihe :

3-[2-(2,4-Dichlorbenzyl)-4-methoxymethyl-1,3-dioxolan-2-yl]-pyridin,
3-[2-(2,4-Dichlorbenzyl)-4-methyl-1,3-dioxolan-2-yl]-pyridin,
3-[2-(2,4-Dichlorbenzyl)-4-chlormethyl-1,3-dioxolan-2-yl]-pyridin,
3-[2-(2,4-Dichlorbenzyl)-1,3-dioxan-2-yl]-pyridin,
3-[2-(2,4-Dichlorbenzyl)-4-hydroxymethyl-1,3-dioxolan-2-yl]-pyridin,
3-[2-(2,4-Dichlorbenzyl)-4-ethyl-1,3-dioxolan-2-yl]-pyridin.

9. Verfahren zur Herstellung von Verbindungen der Formel I, gekennzeichnet,

A) durch Ketalisierungsreaktion eines Ketons der Formel II in Gegenwart einer Säure

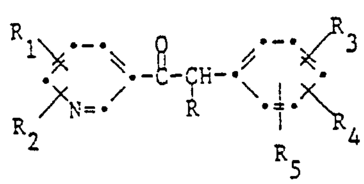

(II)

mit einem Alkohol V—OH oder U—OH, mit einem Diol der Formel HO—U—V—OH oder mit einem Orthocarbonsäureester R'—C(OV)$_3$ oder R'—C(OU)$_3$ oder durch Umketalisierung des erhaltenen Ketals mit einem Ueberschuss des entsprechenden Alkanols oder Diols, dabei haben die Substituenten R bis $R_5$, U und V in Formel II sowie in den Alkoholen und Diolen, die unter Formel I angegebene Bedeutung und R' steht vorzugsweise für einen Niederalkylrest ; oder

E) durch Reaktion einer Verbindung der Formel I'

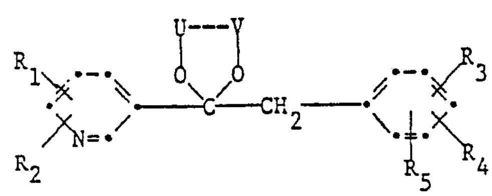

mit einer Verbindung der Formel IX

R—X (IX)

wobei die Substituenten R bis $R_5$ sowie U und V in den Formeln I' und IX wie unter Formel I definiert sind und X für eine übliche Abgangsgruppe steht.

10. Verfahren zur Herstellung von Verbindungen der Formel I, gekennzeichnet,

B) durch Reaktion einer Verbindung der Formel III

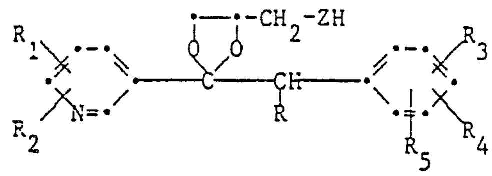

(III)

mit einer reaktionsfähigen, zur O-Alkylierung oder S-Alkylierung geeigneten Verbindung der Formel (IV)

$$R_h—X \qquad (IV)$$

wobei die Substituenten R bis $R_5$, $R_h$ und Z in den Formeln III und IV, die unter Formel I angegebenen Bedeutungen haben und X für eine der üblichen Abgangsgruppen steht ; oder

C) durch Reaktion eines Ketals der Formel V

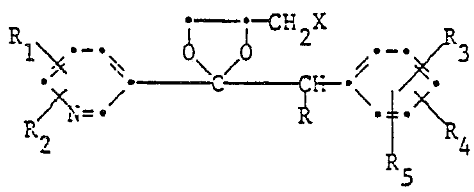

(V)

mit einer Verbindung der Formel VI

$$R_h—Z—M \qquad (VI)$$

wobei die Substituenten R bis $R_5$, $R_h$ und Z wie unter Formel I definiert sind, X die in Variante B angegebenen Bedeutungen hat und M für Wasserstoff oder vorzugsweise ein Metall-, insbesondere ein Alkali-Metallatom steht ; oder

D) durch Kondensations-Reaktion eines Alkohols der Formel VII mit einem Alkanol der Formel VIII

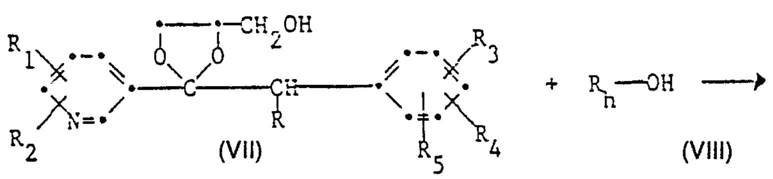

(VII) (VIII) (I)

wobei die Substituenten R bis $R_5$ und $R_h$ wie unter Formel I definiert sind.

11. Mikrobizides Mittel zur Bekämpfung oder Verhütung eines Befalls durch Mikroorganismen, dadurch gekennzeichnet, dass es neben üblichen Zusatzstoffen als mindestens eine aktive Komponente eine Verbindung der Formel I gemäss Anspruch 1 enthält.

12. Mittel gemäss Anspruch 10, dadurch gekennzeichnet, dass es als mindestens eine aktive Komponente eine Verbindung der Formel I gemäss Anspruch 2 enthält.

13. Mittel zur Bekämpfung von Mikroorganismen gemäss Anspruch 10, dadurch gekennzeichnet, dass es als mindestens eine aktive Komponente eine Verbindung der Formel I gemäss Anspruch 3 enthält.

14. Mittel zur Bekämpfung von Mikroorganismen gemäss Anspruch 10, dadurch gekennzeichnet, dass es als mindestens eine aktive Komponente eine Verbindung der Formel I gemäss einem der Ansprüche 4 bis 8 enthält.

15. Verfahren zur Bekämpfung oder Verhütung eines Befalls von Kulturpflanzen durch phytopathogene Mikroorganismen, dadurch gekennzeichnet, dass man eine Verbindung der Formel I gemäss einem der Ansprüche 1 bis 8 auf die Pflanze oder deren Standort appliziert.

**Patentansprüche** (für den Vertragsstaat AT)

1. Mikrobizides Mittel zur Bekämpfung eines Befalls durch Mikroorganismen, dadurch gekennzeichnet, dass es neben üblichen Zuschlagstoffen als mindestens eine aktive Komponente eine Verbindung der Formel I

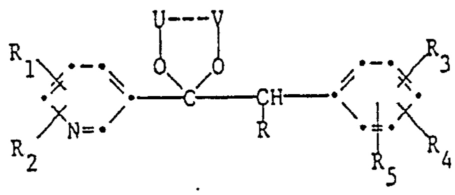

(I)

enthält, worin

R für Wasserstoff, $C_1$-$C_6$-Alkyl, $C_3$-$C_6$-Cycloalkyl, Phenyl, Benzyl, durch $C_1$-$C_4$-Alkyl, Halogen, $C_1$-$C_4$-Alkoxy, $NO_2$ und/oder $CF_3$ substituiertes Phenyl oder durch $C_1$-$C_4$-Alkyl, Halogen, $C_1$-$C_4$-Alkoxy, $NO_2$ und/oder $CF_3$ substituiertes Benzyl steht :

$R_1$ und $R_2$ unabhängig voneinander für Wasserstoff, $C_1$-$C_4$-Alkyl, Halogen oder $C_1$-$C_4$-Alkoxy stehen ;

$R_3$, $R_4$ und $R_5$ unabhängig voneinander Wasserstoff, Halogen, $CF_3$, $NO_2$, $C_1$-$C_4$-Alkyl, $C_1$-$C_4$-Alkoxy, Phenyl oder durch $C_1$-$C_4$-Alkyl, $C_1$-$C_4$-Alkoxy, Halogen, $NO_2$ und/oder $CF_3$ substituiertes Phenyl bedeuten ;

U und V unabhängig voneinander für $C_1$-$C_6$-Alkyl oder durch Halogen oder $C_1$-$C_4$-Alkoxy substituiertes Alkyl stehen oder zusammen eine der folgenden Alkylenbrücken

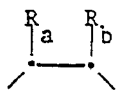 , 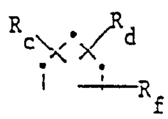 oder 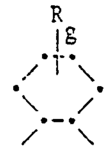 bilden, wobei

$R_a$ für Wasserstoff, $C_1$-$C_{12}$-Alkyl, ein- oder mehrfach durch Halogen substituiertes $C_1$-$C_{12}$-Alkyl, Phenyl, ein- oder mehrfach durch Halogen und/oder $C_1$-$C_4$-Alkyl substituiertes Phenyl oder für die Gruppe $—CH_2—Z—R_h$ steht ;

$R_b$ für $C_1$-$C_{12}$-Alkyl, ein- oder mehrfach durch Halogen substituiertes $C_1$-$C_{12}$-Alkyl, Phenyl, ein- oder mehrfach durch Halogen und/oder $C_1$-$C_4$-Alkyl substituiertes Phenyl oder für die Gruppe $—CH_2—Z—R_h$ steht ;

wobei Z Sauerstoff oder Schwefel bedeutet und

$R_h$ für Wasserstoff, $C_1$-$C_8$-Alkyl, ein durch $C_1$-$C_4$-Alkoxy substituiertes $C_1$-$C_8$-Alkyl, $C_3$-$C_4$-Alkenyl, 2-Propinyl, 3-Halogen-2-propinyl, Phenyl, ein durch Halogen, $C_1$-$C_4$-Alkyl, $C_1$-$C_4$-Alkoxy, $NO_2$ und/oder $CF_3$ substituiertes Phenyl, Benzyl oder durch Halogen, $C_1$-$C_4$-Alkyl, $C_1$-$C_4$-Alkoxy, $NO_2$ und/oder $CF_3$ substituiertes Benzyl steht ;

$R_c$, $R_d$ und $R_f$ unabhängig voneinander Wasserstoff oder $C_1$-$C_4$-Alkyl bedeuten ; und

$R_g$ für Wasserstoff oder $C_1$-$C_4$-Alkyl steht ; unter Einschluss ihrer Säureadditionssalze.

2. Mittel nach Anspruch 1, enthaltend eine Verbindung der Formel I, worin R für Wasserstoff oder Methyl steht ; $R_1$ und $R_2$ unabhängig voneinander für Wasserstoff, Methyl, Ethyl, Fluor, Chlor, Brom oder Methoxy stehen ; $R_3$, $R_4$ und $R_5$ unabhängig voneinander für Wasserstoff, Fluor, Chlor, Brom, $CF_3$, $NO_2$, Methyl oder Methoxy stehen ; U und V unabhängig voneinander $C_1$-$C_3$-Alkyl bedeuten oder zusammen folgende Alkylenbrücke

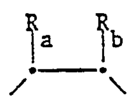

bilden, wobei $R_a$ für Wasserstoff, $C_1$-$C_4$-Alkyl, Phenyl, ein- bis dreifach durch Fluor, Chlor, Brom und/oder Methyl substituiertes Phenyl oder die Gruppe $—CH_2—Z—R_h$ steht ; $R_b$ für $C_1$-$C_4$-Alkyl, ein- bis dreifach durch Fluor, Chlor und/oder Brom substituiertes $C_1$-$C_4$-Alkyl, Phenyl, ein- bis dreifach durch Fluor, Chlor, Brom und/oder Methyl substituiertes Phenyl oder die Gruppe $—CH_2—Z—R_h$ steht ; wobei Z für Sauerstoff oder Schwefel steht ; $R_h$ Wasserstoff, $C_1$-$C_6$-Alkyl, ein durch $C_1$-$C_4$-Alkoxy substituiertes $C_1$-$C_3$-Alkyl, $C_3$-$C_4$-Alkenyl, 2-Propinyl, 3-Halogen-2-propinyl, Phenyl, ein durch Fluor, Chlor, Brom, Methyl, Methoxy, Nitro und/oder $CF_3$ substituiertes Phenyl, Benzyl oder ein durch Fluor, Chlor, Brom, Methyl, Methoxy, $NO_2$ und/oder $CF_3$ substituiertes Benzyl bedeutet ; unter Einschluss ihrer Säureadditionssalze.

3. Mittel nach Anspruch 2, enthaltend eine Verbindung der Formel I, worin R für Wasserstoff steht ; $R_1$ und $R_2$ unabhängig voneinander für Wasserstoff, Methyl, Fluor, Chlor, Brom oder Methoxy stehen ; $R_3$, $R_4$ und $R_5$ unabhängig voneinander für Wasserstoff, Fluor, Chlor, Brom, $CF_3$, $NO_2$, Methyl oder Methoxy stehen ; U und V zusammen folgende Alkylenbrücke

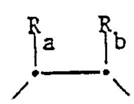

bilden, wobei $R_a$ für Wasserstoff oder Methyl steht ; $R_b$ für $C_1$-$C_4$-Alkyl, durch Fluor, Chlor oder Brom substituiertes $C_1$-$C_4$-Alkyl, Phenyl, ein- bis dreifach durch Fluor, Chlor, Brom und/oder Methyl substituiertes Phenyl oder die Gruppe —$CH_2$—Z—$R_h$ steht ; wobei Z für Sauerstoff oder Schwefel steht ; $R_h$ Wasserstoff, $C_1$-$C_4$-Alkyl, ein durch $C_1$-$C_4$-Alkoxy substituiertes $C_1$-$C_2$-Alkyl, $C_3$-$C_4$-Alkenyl, 2-Propinyl, 3-Halogen-2-propinyl, Phenyl, ein durch Fluor, Chlor, Brom, Methyl, Methoxy, Nitro und/oder $CF_3$ substituiertes Phenyl, Benzyl oder ein durch Fluor, Chlor, Brom, Methyl, Methoxy, Nitro und/oder $CF_3$ substituiertes Benzyl bedeutet.

4. Mittel nach Anspruch 1, enthaltend eine Verbindung der Formel I, worin R für Wasserstoff oder Methyl steht ; $R_1$ und $R_2$ unabhängig voneinander für Wasserstoff, Methyl, Ethyl, Fluor, Chlor, Brom oder Methoxy stehen ; $R_3$, $R_4$ und $R_5$ unabhängig voneinander für Wasserstoff, Fluor, Chlor, Brom, $CF_3$, $NO_2$, Methyl oder Methoxy stehen ; U und V unabhängig voneinander $C_1$-$C_3$-Alkyl bedeuten oder zusammen folgende Alkylenbrücke

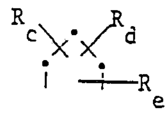

bilden, wobei $R_c$, $R_d$ und $R_e$ unabhängig voneinander für Wasserstoff oder $C_1$-$C_4$-Alkyl stehen und die Summe der Kohlenstoffatome in $R_c$, $R_d$ und $R_e$ die Zahl 6 nicht übersteigt ; unter Einschluss ihrer Säureadditionssalze.

5. Mittel nach Anspruch 4, enthaltend eine Verbindung der Formel I, worin R für Wasserstoff steht ; $R_1$ und $R_2$ unabhängig voneinander für Wasserstoff, Methyl, Fluor, Chlor, Brom oder Methoxy stehen ; $R_3$, $R_4$ und $R_5$ unabhängig voneinander für Wasserstoff, Fluor, Chlor, Brom, $CF_3$, $NO_2$, Methyl oder Methoxy stehen ; U und V zusammen folgende Alkylenbrücke

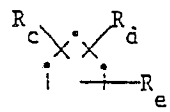

bilden, wobei $R_c$ Wasserstoff bedeutet und $R_d$ und $R_e$ unabhängig voneinander für Wasserstoff, Methyl oder Ethyl stehen.

6. Mittel nach Anspruch 1, enthaltend eine Verbindung der Formel I, worin R für Wasserstoff oder Methyl steht ; $R_1$ und $R_2$ unabhängig voneinander für Wasserstoff, Methyl, Ethyl, Fluor, Chlor, Brom oder Methoxy stehen ; $R_3$, $R_4$ und $R_5$ unabhängig voneinander für Wasserstoff, Fluor, Chlor, Brom, $CF_3$, $NO_2$, Methyl oder Methoxy stehen, U und V unabhängig voneinader $C_1$-$C_3$-Alkyl bedeuten oder zusammen folgende Alkylenbrücke

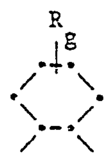

bilden, wobei $R_g$ für Wasserstoff oder $C_1$-$C_4$-Alkyl steht ; unter Einschluss ihrer Säureadditionssalze.

7. Mittel nach Anspruch 6, enthaltend eine Verbindung der Formel I, worin R für Wasserstoff steht ; $R_1$ und $R_2$ unabhängig voneinander für Wasserstoff, Methyl, Fluor, Chlor, Brom oder Methoxy stehen ; $R_3$, $R_4$ und $R_5$ unabhängig voneinander für Wasserstoff, Fluor, Chlor, Brom, $CF_3$, $NO_2$, Methyl oder Methoxy stehen ; U und V zusammen folgende Alkylenbrücke

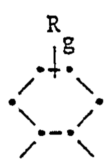

bilden, wobei $R_g$ für Wasserstoff, Methyl oder Ethyl steht.

8. Mittel nach Anspruch 1, enthaltend eine Verbindung der Formel I in Form ihres Isomerengemisches oder in optisch reiner Form, ausgewählt aus der Reihe :

3-[2-(2,4-Dichlorbenzyl)-4-methoxymethyl-1,3-dioxolan-2-yl]-pyridin,
3-[2-(2,4-Dichlorbenzyl)-4-methyl-1,3-dioxolan-2-yl]-pyridin,
3-[2-(2,4-Dichlorbenzyl)-4-chlormethyl-1,3-dioxolan-2-yl]-pyridin,
3-[2-(2,4-Dichlorbenzyl)-1,3-dioxan-2-yl]-pyridin,
3-[2-(2,4-Dichlorbenzyl)-4-hydroxymethyl-1,3-dioxolan-2-yl]-pyridin,
3-[2-(2,4-Dichlorbenzyl)-4-ethyl-1,3-dioxolan-2-yl]-pyridin.

9. Verfahren zur Herstellung von Verbindungen der Formel I, gekennzeichnet,

A) durch Ketalisierungsreaktion eines Ketons der Formel II in Gegenwart einer Säure

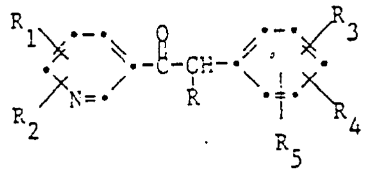

(II)

mit einem Alkohol V—OH oder U—OH, mit einem Diol der Formel HO—U—V—OH oder mit einem Orthocarbonsäureester R'—C(OV)$_3$ oder R'—C(OU)$_3$ oder durch Umketalisierung des erhaltenen Ketals mit einem Ueberschuss des entsprechenden Alkanols oder Diols, dabei haben die Substituenten R bis $R_5$, U und V in Formel II sowie in den Alkoholen und Diolen, die unter Formel I angegebene Bedeutung und R' steht vorzugsweise für einen Niederalkylrest ; oder

E) durch Reaktion einer Verbindung der Formel I'

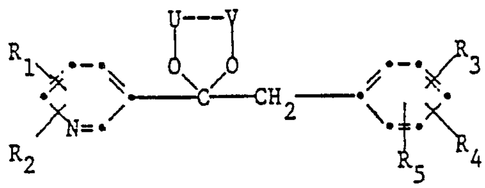

(I')

kann durch Reaktion mit einer Verbindung der Formel IX

R—X (IX)

wobei die Substituenten R bis $R_5$ sowie U und V in den Formeln I' und IX wie unter Formel I definiert sind und X für eine übliche Abgangsgruppe steht.

10. Verfahren zur Herstellung von Verbindungen der Formel I, gekennzeichnet,

B) durch Reaktion einer Verbindung der Formel III

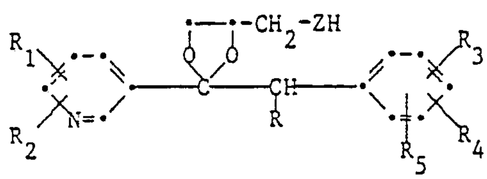

(III)

mit einer reaktionsfähigen, zur O-Alkylierung oder S-Alkylierung geeigneten Verbindung der Formel (IV)

$R_h$—X (IV)

wobei die Substituenten R bis $R_5$, $R_h$ und Z in den Formeln III und IV, die unter Formel I angegebenen Bedeutungen haben und X für eine der üblichen Abgangsgruppen steht, oder

C) durch Reaktion eines Ketals der Formel V

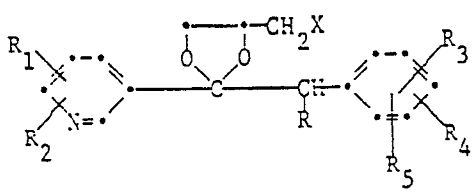

(V)

mit einer Verbindung der Formel VI

$R_h$—Z—M (VI)

wobei die Substituenten R bis $R_5$, $R_h$ und Z wie unter Formel I definiert sind, X die in Variante B angegebenen Bedeutungen hat und M für Wasserstoff oder vorzugsweise ein Metall-, insbesondere ein Alkali-Metallatom steht ; oder

D) durch Kondensations-Reaktion eines Alkohols der Formel VII mit einem Alkanol der Formel VIII

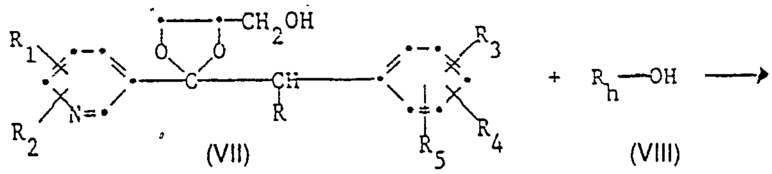

(VII) (VIII) (I)

wobei die Substituenten R bis $R_5$ und $R_h$ wie unter Formel I definiert sind ;

11. Verfahren zur Herstellung eines agrochemischen Mittels, dadurch gekennzeichnet, dass man mindestens eine Verbindung der Formel I mit geeigneten festen oder flüssigen Zusatzstoffen und Tensiden innig vermischt.

12. Verfahren zur Bekämpfung oder Verhütung eines Befalls von Kulturpflanzen durch phytopathogene Mikroorganismen, dadurch gekennzeichnet, dass man eine Verbindung der Formel I auf die Planze oder deren Standort appliziert.

**Claims** (for the Contracting States : BE, DE, FR, IT, LU, SE, CH, LI)

1. A compound of the general formula I

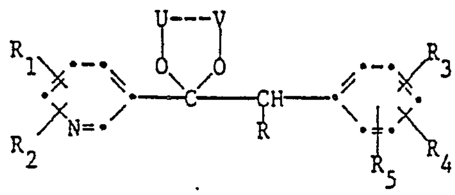

(I)

wherein

R is hydrogen, $C_1$-$C_6$ alkyl, $C_3$-$C_6$ cycloalkyl, phenyl, benzyl, phenyl substituted by $C_1$-$C_4$ alkyl, halogen, $C_1$-$C_4$ alkoxy, $NO_2$ and/or $CF_3$, or benzyl substituted by $C_1$-$C_4$ alkyl, halogen, $C_1$-$C_4$ alkoxy, $NO_2$ and/or $CF_3$,

$R_1$ and $R_2$ independently of one another are each hydrogen, $C_1$-$C_4$ alkyl, halogen or $C_1$-$C_4$ alkoxy,

$R_3$, $R_4$ and $R_5$ independently of one another are each hydrogen, halogen, $CF_3$, $NO_2$, $C_1$-$C_4$ alkyl, $C_1$-$C_4$ alkoxy, phenyl, or phenyl substituted by $C_1$-$C_4$ alkyl, $C_1$-$C_4$ alkoxy, halogen, $NO_2$ and/or $CF_3$,

U and V independently of one another are each $C_1$-$C_6$ alkyl, or alkyl substituted by halogen or $C_1$-$C_4$ alkoxy, or together form one of the following alkylene bridges

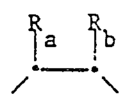 , 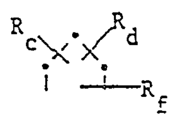 or 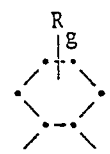 in which

$R_a$ is hydrogen, $C_1$-$C_{12}$ alkyl, $C_1$-$C_{12}$ alkyl which is mono- or polysubstituted by halogen, or is phenyl, phenyl which is mono- or polysubstituted by halogen and/or $C_1$-$C_4$ alkyl, or is the group $-CH_2-Z-R_h$,

$R_b$ is $C_1$-$C_{12}$ alkyl, $C_1$-$C_{12}$ alkyl which is mono- or polysubstituted by halogen, or is phenyl, phenyl which is mono- or polysubstituted by halogen and/or $C_1$-$C_4$ alkyl, or is the group $-CH_2-Z-R_h$, in which

Z is oxygen or sulfur, and

$R_h$ is hydrogen, $C_1$-$C_8$ alkyl, $C_1$-$C_8$ alkyl which is substituted by $C_1$-$C_4$-alkoxy, or is $C_3$-$C_4$ alkenyl, 2-propynyl, 3-halo-2-propynyl, phenyl, phenyl which is substituted by halogen, $C_1$-$C_4$ alkyl, $C_1$-$C_4$ alkoxy, $NO_2$ and/or $CF_3$, or is benzyl, or benzyl which is substituted by halogen, $C_1$-$C_4$ alkyl, $C_1$-$C_4$ alkoxy, $NO_2$ and/or $CF_3$,

$R_c$, $R_d$ and $R_f$ independently of one another are each hydrogen or $C_1$-$C_4$-alkyl, and

$R_g$ is hydrogen or $C_1$-$C_4$ alkyl ;

including the acid addition salts thereof.

2. A compound of the formula I according to claim 1, wherein R is hydrogen or methyl, $R_1$ and $R_2$ independently of one another are each hydrogen, methyl, ethyl, fluorine, chlorine, bromine or methoxy, $R_3$, $R_4$ and $R_5$ independently of one another are each hydrogen, fluorine, chlorine, bromine, $CF_3$, $NO_2$, methyl or methoxy, U and V independently of one another are each $C_1$-$C_3$ alkyl, or together form the following alkylene bridge

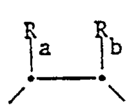

in which $R_a$ is hydrogen, $C_1$-$C_4$ alkyl, phenyl, or phenyl which is mono- to trisubstituted by fluorine, chlorine, bromine and/or methyl, or is the group —$CH_2$—Z—$R_h$, $R_b$ is $C_1$-$C_4$ alkyl, or $C_1$-$C_4$ alkyl which is mono- to trisubstituted by fluorine, chlorine and/or bromine, or is phenyl, or phenyl which is mono- to trisubstituted by fluorine, chlorine, bromine and/or methyl, or is the group —$CH_2$—Z—$R_h$ in which Z is oxygen or sulfur, and $R_h$ is hydrogen, $C_1$-$C_6$ alkyl, $C_1$-$C_3$ alkyl which is substituted by $C_1$-$C_4$ alkoxy, or is $C_3$-$C_4$ alkenyl, 2-propynyl, 3-halo-2-propynyl, phenyl, or phenyl which is substituted by fluorine, chlorine, bromine, methyl, methoxy, nitro and/or $CF_3$, or is benzyl, or benzyl which is substituted by fluorine, chlorine, bromine, methyl, methoxy, $NO_2$ and/or $CF_3$ ; including the acid addition salts thereof.

3. A compound of the formula I according to claim 2, wherein R is hydrogen, $R_1$ and $R_2$ independently of one another are each hydrogen, methyl, fluorine, chlorine, bromine or methoxy, $R_3$, $R_4$ and $R_5$ independently of one another are each hydrogen, fluorine, chlorine, bromine, $CF_3$, $NO_2$, methyl or methoxy, U and V together form the following alkylene bridge

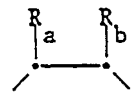

in which $R_a$ is hydrogen or methyl, and $R_b$ is $C_1$-$C_4$ alkyl, or $C_1$-C4 alkyl substituted by fluorine, chlorine or bromine, or is phenyl, or phenyl which is mono- to trisubstituted by fluorine, chlorine, bromine and/or methyl, or is the group —$CH_2$—Z—$R_h$, in which Z is oxygen or sulfur, and $R_h$ is hydrogen, $C_1$-$C_4$ alkyl, or $C_1$-$C_2$ alkyl substituted by $C_1$-$C_4$ alkoxy, or is $C_3$-$C_4$ alkenyl, 2-propynyl, 3-halo-2-propynyl, phenyl, or phenyl which is substituted by fluorine, chlorine, bromine, methyl, methoxy, nitro and/or $CF_3$, or is benzyl, or benzyl which is substituted by fluorine, chlorine, bromine, methyl, methoxy, nitro and/or $CF_3$.

4. A compound of the formula I according to claim 1, wherein R is hydrogen or methyl, $R_1$ and $R_2$ independently of one another are each hydrogen, methyl, ethyl, fluorine, chlorine, bromine or methoxy, $R_3$, $R_4$ and $R_5$ independently of one another are each hydrogen, fluorine, chlorine, bromine $CF_3$, $NO_2$, methyl or methoxy, U and V independently of one another are each $C_1$-$C_3$ alkyl, or together form the following alkylene bridge

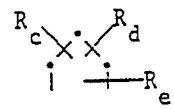

in which $R_c$, $R_d$ and $R_e$ independently of one another are each hydrogen or $C_1$-$C_4$ alkyl, and the total number of carbon atoms in $R_c$, $R_d$ and $R_e$ does not exceed 6 ; including the acid addition salts thereof.

5. A compound of the formula I according to claim 4, wherein R is hydrogen, $R_1$ and $R_2$ independently of one another are each hydrogen, methyl, fluorine, chlorine, bromine or methoxy, $R_3$, $R_4$ and $R_5$ independently of one another are each hydrogen, fluorine, chlorine, bromine, $CF_3$, $NO_2$, methyl or methoxy, U and V together form the following alkylene bridge

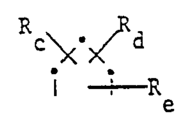

in which $R_c$ is hydrogen, and $R_d$ and $R_e$ independently of one another are each hydrogen, methyl or ethyl.

6. A compound of the formula I according to claim 1, wherein R is hydrogen or methyl, $R_1$ and $R_2$ independently of one another are each hydrogen, methyl, ethyl, fluorine, chlorine, bromine or methoxy, $R_3$, $R_4$ and $R_5$ independently of one another are each hydrogen, fluorine, chlorine, bromine, $CF_3$, $NO_2$, methyl or methoxy, U and V independently of one another are each $C_1$-$C_3$ alkyl, or together form the following alkylene bridge

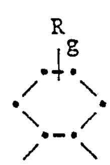

in which $R_g$ is hydrogen or $C_1$-$C_4$ alkyl ; including the acid addition salts thereof.

7. A compound of the formula I according to claim 6, wherein R is hydrogen, $R_1$ and $R_2$ independently of one another are each hydrogen, methyl, fluorine, chlorine, bromine or methoxy, $R_3$, $R_4$ and $R_5$ independently of one another are each hydrogen, fluorine, chlorine, bromine, $CF_3$, $NO_2$, methyl or methoxy, U and V together form the following alkylene bridge

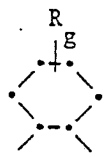

in which $R_g$ is hydrogen, methyl or ethyl.

8. A compound of the formula I according to claim 1, as a mixture of the isomers thereof or in the optically pure form, selected from the series :

3-[2-(2,4-dichlorobenzyl)-4-methoxymethyl-1,3-dioxolan-2-yl]-pyridine,
3-[2-(2,4-dichlorobenzyl)-4-methyl-1,3-dioxolan-2-yl]-pyridine,
3-[2-(2,4-dichlorobenzyl)-4-chloromethyl-1,3-dioxolan-2-yl]-pyridine,
3-[2-(2,4-dichlorobenzyl)-1,3-dioxan-2-yl]-pyridine,
3-[2-(2,4-dichlorobenzyl)-4-hydroxymethyl-1,3-dioxolan-2-yl]-pyridine,
3-[2-(2,4-dichlorobenzyl)-4-ethyl-1,3-dioxolan-2-yl]-pyridine.

9. A process for producing a compound of the formula I
A) by a ketalisation reaction of a ketone of the formula II

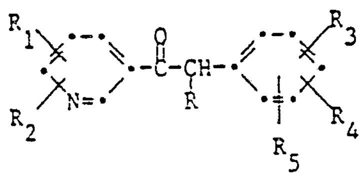

(II)

in the presence of an acid, with an alcohol V—OH or U—OH, with a diol of the formula HO—U—V—OH or with an orthocarboxylic acid ester R'—C(OV)$_3$ or R'—C(OU)$_3$, or by transketalisation of the resulting ketal with an excess of the corresponding alkanol or diol, the substituents R to $R_5$, U and V in the formula II, as well as in the alcohols and diols, having the meanings defined under the formula I, and R' being preferably a lower alkyl radical ; or
E) by reaction of a compound of the formula I'

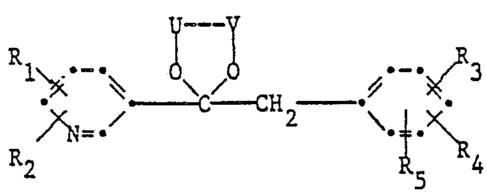

(I')

with a compound of the formula IX

R—X (IX),

the substituents R to $R_5$ and U and V in the formulae I' and IX having the meanings defined under the formula I, and X being a customary removable group.

10. A process for producing a compound of the formula I
B) by reaction of a compound of the formula III

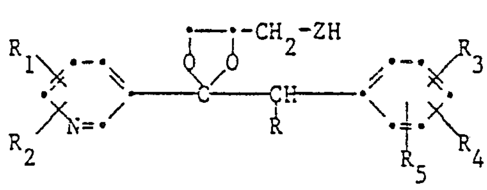

(III)

with a reactive compound of the formula IV suitable for O-alkylation or S-alkylation

$R_h$—X (IV),

the substituents R to $R_5$, $R_h$ and Z in the formulae III and IV having the meanings defined under formula I,

and X being one of the customary removable groups ; or

C) by reaction of a ketal of the formula V

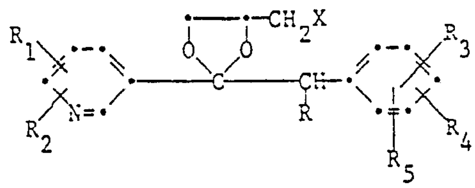

(V)

with a compound of the formula VI

$R_h$—Z—M (VI),

the substituents R to $R_5$, $R_h$ and Z having the meanings defined under the formula I, X having the meanings given in variant B, and M being hydrogen or preferably a metal atom, especially an alkali metal atom ; or

D) by a condensation reaction of an alcohol of the formula VII with an alkanol of the formula VIII

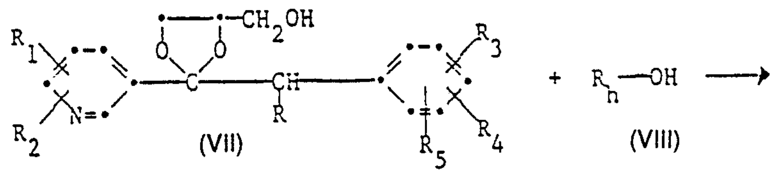

(VII) (VIII) (I)

the substituents R to $R_5$ and $R_h$ having the meanings defined under the formula I.

11. A microbicidal agent for controlling or preventing an infestation by microorganisms, which, in addition to customary additives, contains, as at least one active component, a compound of the formula I according to claim 1.

12. An agent according to claim 10, which contains, as at least one active component, a compound of the formula I according to claim 2.

13. An agent for controlling microorganisms according to claim 10, which contains, as at least one active component, a compound of the formula I according to claim 3.

14. An agent for controlling microorganisms according to claim 10, which contains, as at least one active component, a compound of the formula I according to any one of claims 4 to 8.

15. A process for controlling or preventing an infestation of cultivated plants by phytopathogenic microorganisms, which process comprises applying to the plants or to the locus thereof a compound of the formula I according to any one of claims 1 to 8.

**Claims** (for the Contracting State AT)

1. A microbicidal agent for controlling or preventing an infestation by microorganisms, which, in addition to customary additives, contains, as at least one active component, a compound of the formula I

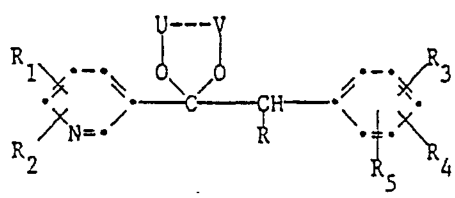

(I)

wherein

R is hydrogen, $C_1$-$C_6$ alkyl, $C_3$-$C_6$ cycloalkyl, phenyl, benzyl, phenyl substituted by $C_1$-$C_4$ alkyl, halogen, $C_1$-$C_4$ alkoxy, $NO_2$ and/or $CF_3$, or benzyl substituted by $C_1$-$C_4$ alkyl, halogen, $C_1$-$C_4$ alkoxy, $NO_2$ and/or $CF_3$,

$R_1$ and $R_2$ independently of one another are each hydrogen, $C_1$-$C_4$ alkyl, halogen or $C_1$-$C_4$ alkoxy,

$R_3$, $R_4$ and $R_5$ independently of one another are each hydrogen, halogen, $CF_3$, $NO_2$, $C_1$-$C_4$ alkyl, $C_1$-$C_4$ alkoxy, phenyl, or phenyl substituted by $C_1$-$C_4$ alkyl, $C_1$-$C_4$ alkoxy, halogen, $NO_2$ and/or $CF_3$,

U and V independently of one another are each $C_1$-$C_6$ alkyl, or alkyl substituted by halogen or $C_1$-$C_4$ alkoxy, or together form one of the following alkylene bridges

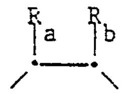 , 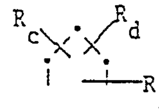 or 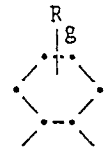 in which

$R_a$ is hydrogen, $C_1$-$C_{12}$ alkyl, $C_1$-$C_{12}$ alkyl which is mono- or polysubstituted by halogen, or is phenyl, phenyl which is mono- or polysubstituted by halogen and/or $C_1$-$C_4$ alkyl, or is the group $—CH_2—Z—R_h$,

$R_b$ is $C_1$-$C_{12}$ alkyl, $C_1$-$C_{12}$ alkyl which is mono- or polysubstituted by halogen, or is phenyl, phenyl which is mono- or polysubstituted by halogen and/or $C_1$-$C_4$ alkyl, or is the group $—CH_2—Z—R_h$, in which

Z is oxygen or sulfur, and

$R_h$ is hydrogen, $C_1$-$C_8$ alkyl, $C_1$-$C_8$ alkyl which is substituted by $C_1$-$C_4$ alkoxy, or is $C_3$-$C_4$ alkenyl, 2-propynyl, 3-halo-2-propynyl, phenyl, phenyl which is substituted by halogen, $C_1$-$C_4$ alkyl, $C_1$-$C_4$ alkoxy, $NO_2$ and/or $CF_3$, or is benzyl, or benzyl which is substituted by halogen, $C_1$-$C_4$ alkyl, $C_1$-$C_4$ alkoxy, $NO_2$ and/or $CF_3$,

$R_c$, $R_d$ and $R_f$ independently of one another are each hydrogen or $C_1$-$C_4$ alkyl, and

$R_g$ is hydrogen or $C_1$-$C_4$ alkyl ;

including the acid addition salts thereof.

2. An agent according to claim 1, containing a compound of the formula I wherein R is hydrogen or methyl, $R_1$ and $R_2$ independently of one another are each hydrogen, methyl, ethyl, fluorine, chlorine, bromine or methoxy, $R_3$, $R_4$ and $R_5$ independently of one another are each hydrogen, fluorine, chlorine, bromine, $CF_3$, $NO_2$, methyl or methoxy, U and V independently of one another are each $C_1$-$C_3$ alkyl, or together form the following alkylene bridge

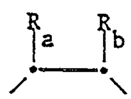

in which $R_a$ is hydrogen, $C_1$-$C_4$ alkyl, phenyl, or phenyl which is mono- to trisubstituted by fluorine, chlorine, bromine and/or methyl, or is the group $—CH_2—Z—R_h$, $R_b$ is $C_1$-$C_4$ alkyl, or $C_1$-$C_4$ alkyl which is mono- to trisubstituted by fluorine, chlorine and/or bromine, or is phenyl, or phenyl which is mono- to trisubstituted by fluorine, chlorine, bromine and/or methyl, or is the group $—CH_2—Z—R_h$ in which Z is oxygen or sulfur, and $R_h$ is hydrogen, $C_1$-$C_6$ alkyl, $C_1$-$C_3$ alkyl which is substituted by $C_1$-$C_4$ alkoxy, or is $C_3$-$C_4$ alkenyl, 2-propynyl, 3-halo-2-propynyl, phenyl, or phenyl which is substituted by fluorine, chlorine, bromine, methyl, methoxy, nitro and/or $CF_3$, or is benzyl, or benzyl which is substituted by fluorine, chlorine, bromine, methyl, methoxy, $NO_2$ and/or $CF_3$ ; including the acid addition salts thereof.

3. An agent according to claim 2, containing a compound of the formula I wherein R is hydrogen, $R_1$ and $R_2$ independently of one another are each hydrogen, methyl, fluorine, chlorine, bromine or methoxy, $R_3$, $R_4$ and $R_5$ independently of one another are each hydrogen, fluorine, chlorine, bromine, $CF_3$, $NO_2$, methyl or methoxy, U and V together form the following alkylene bridge

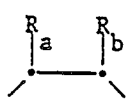

in which $R_a$ is hydrogen or methyl, and $R_b$ is $C_1$-$C_4$ alkyl, or $C_1$-$C_4$ alkyl substituted by fluorine, chlorine or bromine, or is phenyl, or phenyl which is mono- to trisubstituted by fluorine, chlorine, bromine and/or methyl, or is the group $—CH_2—Z—R_h$, in which Z is oxygen or sulfur, and $R_h$ is hydrogen, $C_1$-$C_4$ alkyl, or $C_1$-$C_2$ alkyl substituted by $C_1$-$C_4$ alkoxy, or is $C_3$-$C_4$ alkenyl, 2-propynyl, 3-halo-2-propynyl, phenyl, or phenyl which is substituted by fluorine, chlorine, bromine, methyl, methoxy, nitro and/or $CF_3$, or is benzyl, or benzyl which is substituted by fluorine, chlorine, bromine, methyl, methoxy, nitro and/or $CF_3$.

4. An agent according to claim 1, containing a compound of the formula I wherein R is hydrogen or methyl, $R_1$ and $R_2$ independently of one another are each hydrogen, methyl, ethyl, fluorine, chlorine, bromine or methoxy, $R_3$, $R_4$ and $R_5$ independently of one another are each hydrogen, fluorine, chlorine, bromine $CF_3$, $NO_2$, methyl or methoxy, U and V independently of one another are each $C_1$-$C_3$ alkyl, or together form the following alkylene bridge

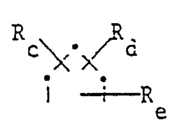

in which $R_c$, $R_d$ and $R_e$ independently of one another are each hydrogen or $C_1$-$C_4$ alkyl, and the total number of carbon atoms in $R_c$, $R_d$ and $R_e$ does not exceed 6 ; including the acid addition salts thereof.

5. An agent according to claim 4, containing a compound of the formula I wherein R is hydrogen, R-

and $R_2$ independently of one another are each hydrogen, methyl, fluorine, chlorine, bromine or methoxy, $R_3$, $R_4$ and $R_5$ independently of one another are each hydrogen, fluorine, chlorine, bromine, $CF_3$, $NO_2$, methyl or methoxy, U and V together form the following alkylene bridge

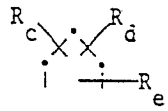

in which $R_c$ is hydrogen, and $R_d$ and $R_e$ independently of one another are each hydrogen, methyl or ethyl.

6. An agent according to claim 1, containing a compound of the formula I wherein R is hydrogen or methyl, $R_1$ and $R_2$ independently of one another are each hydrogen, methyl, ethyl, fluorine, chlorine, bromine or methoxy, $R_3$, $R_4$ and $R_5$ independently of one another are each hydrogen, fluorine, chlorine, bromine, $CF_3$, $NO_2$, methyl or methoxy, U and V independently of one another are each $C_1$-$C_3$ alkyl, or together form the following alkylene bridge

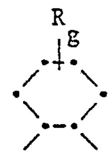

in which $R_g$ is hydrogen or $C_1$-$C_4$ alkyl ; including the acid addition salts thereof.

7. An agent according to claim 6, containing a compound of the formula I wherein R is hydrogen, $R_1$ and $R_2$ independently of one another are each hydrogen, methyl, fluorine, chlorine, bromine or methoxy, $R_3$, $R_4$ and $R_5$ independently of one another are each hydrogen, fluorine, chlorine, bromine, $CF_3$, $NO_2$, methyl or methoxy, U and V together form the following alkylene bridge

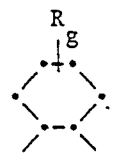

in which $R_g$ is hydrogen, methyl or ethyl.

8. An agent according to claim 1, containing a compound of the formula I in the form of a mixture of the isomers thereof or in the optically pure form, selected from the series :

3-[2-(2,4-dichlorobenzyl)-4-methoxymethyl-1,3-dioxolan-2yl]-pyridine,
3-[2-(2,4-dichlorobenzyl)-4-methyl-1,3-dioxolan-2-yl]-pyridine,
3-[2-(2,4-dichlorobenzyl)-4-chloromethyl-1,3-dioxolan-2-yl]-pyridine,
3-[2-(2,4-dichlorobenzyl)-1,3-dioxan-2-yl]-pyridine,
3-[2-(2,4-dichlorobenzyl)-4-hydroxymethyl-1,3-dioxolan-2-yl]-pyridine,
3-[2-(2,4-dichlorobenzyl)-4-ethyl-1,3-dioxolan-2-yl]-pyridine.

9. A process for producing a compound of the formula I

A) by a ketalisation reaction of a ketone of the formula II

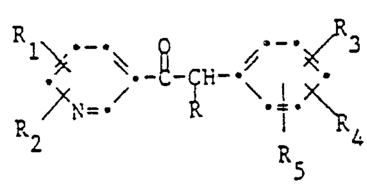

(II)

in the presence of an acid, with an alcohol V—OH or U—OH, with a diol of the formula HO—U—V—OH or with an orthocarboxylic acid ester R'—C(OV)$_3$ or R'—C(OU)$_3$, or by transketalisation of the resulting ketal with an excess of the corresponding alkanol or diol, the substituents R to $R_5$, U and V in the formula II, as well as in the alcohols and diols, having the meanings defined under the formula I, and R' being preferably a lower alkyl radical ; or

E) by reaction of a compound of the formula I'

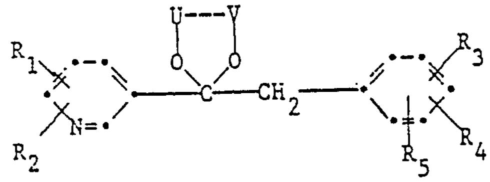

(I')

with a compound of the formula IX

R—X (IX),

the substituents R to $R_5$ and U and V in the formulae I' and IX having the meanings defined under the formula I, and X being a customary removable group.

10. A process for producing a compound of the formula I

B) by reaction of a compound of the formula III

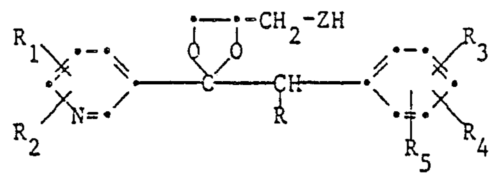

(III)

with a reactive compound of the formula IV suitable for O-alkylation or S-alkylation

$R_h$—X (IV),

the substituents R to $R_5$, $R_h$ and Z in the formulae III and IV having the meanings defined under formula I, and X being one of the customary removable groups ; or

C) by reaction of a ketal of the formula V

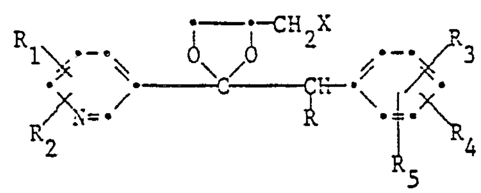

(V)

with a compound of the formula VI

$R_h$—Z—M (VI),

the substituents R to $R_5$, $R_h$ and Z having the meanings defined under the formula I, X having the meanings given in variant B, and M being hydrogen or preferably a metal atom, especially an alkali metal atom ; or

D) by a condensation reaction of an alcohol of the formula VII with an alkanol of the formula VIII

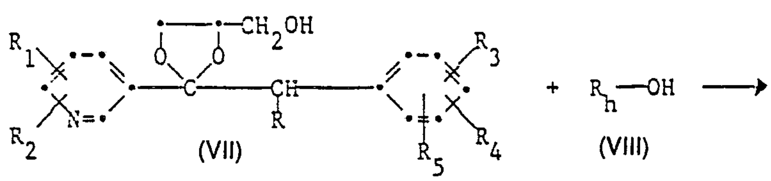

(VII) + (VIII) (I)

the substituents R to $R_5$ and $R_h$ having the meanings defined under the formula I.

11. A process for producing an agrochemical agent, which process comprises intimately mixing a compound of the formula I with suitable solid or liquid additives and surfactants.

12. A process for controlling or preventing an infestation of cultivated plants by phytopathogenic microorganisms, which process comprises applying to the plants or to the locus thereof a compound of the formula I.

**Revendications** (pour les Etats contractants : BE, DE, FR, IT, LU, SE, CH, LI)

1. Composés de formule générale

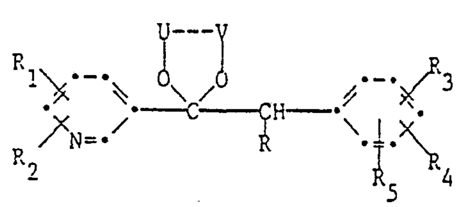

(I)

dans laquelle

R représente l'hydrogène, un groupe alkyle en C 1-C 6, cycloalkyle en C 3-C 6, phényle, benzyle, phényle substitué par des groupes alkyle en C 1-C 4, des halogènes, des groupes alcoxy en C 1-C 4, $NO_2$ et/ou $CF_3$, ou benzyle substitué par des groupes alkyle en C 1-C 4, des halogènes, des groupes alcoxy en C 1-C 4, $NO_2$ et/ou $CF_3$ ;

$R_1$ et $R_2$ représentent chacun, indépendamment l'un de l'autre, l'hydrogène, un groupe alkyle en C 1-C 4, un halogène ou un groupe alcoxy en C 1-C 4 ;

$R_3$, $R_4$ et $R_5$ représentent chacun, indépendamment les uns des autres, l'hydrogène, un halogène, un groupe $CF_3$, $NO_2$, alkyle en C 1-C 4, alcoxy en C 1-C 4, phényle ou phényle substitué par des groupes alkyle en C 1-C 4, alcoxy en C 1-C 4, des halogènes, des groupes $NO_2$ et/ou $CF_3$ ;

U et V représentent chacun, indépendamment l'un de l'autre, un groupe alkyle en C 1-C 6 ou un groupe alkyle substitué par des halogènes ou des groupes alcoxy en C 1-C 4 ou forment ensemble l'un des ponts alkylène suivants

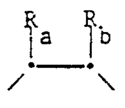 , 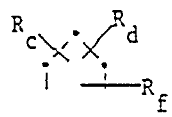 ou 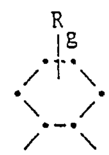

dans lesquels

$R_a$ représente l'hydrogène, un groupe alkyle en C 1-C 12, un groupe alkyle en C 1-C 12 mono- ou poly-substitué par des halogènes, un groupe phényle, un groupe phényle mono- ou poly-substitué par des halogènes et/ou des groupes alkyle en C 1-C 4, ou le groupe $—CH_2—Z—R_h$ ;

$R_b$ représente un groupe alkyle en C 1-C 12, un groupe alkyle en C 1-C 12 mono- ou poly-substitué par des halogènes, un groupe phényle, un groupe phényle mono- ou poly-substitué par des halogènes et/ou des groupes alkyle en C 1-C 4, ou le groupe $—CH_2—Z—R_h$ ;

Z représente l'oxygène ou le soufre,

$R_h$ représente l'hydrogène, un groupe alkyle en C 1-C 8, un groupe alkyle en C 1-C 8 substitué par des groupes alcoxy en C 1-C 4, un groupe alcényle en C 3-C 4, 2-propynyle, 3-halogéno-2-propynyle, phényle, un groupe phényle substitué par des halogènes, des groupes alkyle en C 1-C 4, alcoxy en C 1-C 4, $NO_2$ et/ou $CF_3$, un groupe benzyle ou un groupe benzyle substitué par des halogènes, des groupes alkyle en C 1-C 4, alcoxy en C 1-C 4, $NO_2$ et/ou $CF_3$ ;

$R_c$, $R_d$ et $R_f$ représentent chacun, indépendamment les uns des autres, l'hydrogène ou un groupe alkyle en C 1-C 4 ; et

$R_g$ représente l'hydrogène ou un groupe alkyle en C 1-C 4 ;

y compris leurs sels formés par addition avec des acides.

2. Composés de formule I selon la revendication 1, dans lesquels R représente l'hydrogène ou un groupe méthyle ; $R_1$ et $R_2$ représentent chacun, indépendamment l'un de l'autre, l'hjydrogène, un groupe méthyle, éthyle, le fluor, le chlore, le brome ou un groupe méthoxy ; $R_3$, $R_4$ et $R_5$ représentent chacun, indépendamment les uns des autres, l'hydrogène, le fluor, le chlore, le brome, un groupe $CF_3$, $NO_2$, méthyle ou méthoxy ; U et V représentent chacun, indépendamment l'un de l'autre, un groupe alkyle en C 1-C 3 ou forment ensemble un pont alkylène

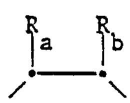

dans lequel $R_a$ représente l'hydrogène, un groupe alkyle en C 1-C 4, un groupe phényle, un groupe phényle mono- à tri-substitué par le fluor, le chlore, le brome et/ou des groupes méthyle, ou le groupe $—CH_2—Z—R_h$ ; $R_b$ représente un groupe alkyle en C 1-C 4, un groupe alkyle en C 1-C 4 mono- à tri-substitué par le fluor, le chlore et/ou le brome, un groupe phényle, un groupe phényle mono- à tri-substitué par le fluor, le chlore, le brome et/ou des groupes méthyle ; ou le groupe $—CH_2—Z—R_h$ ; Z représente l'oxygène ou le soufre ; $R_h$ représente l'hydrogène, un groupe alkyle en C 1-C 6, un groupe alkyle en C 1-C 3 substitué par des groupes alcoxy en C 1-C 4, un groupe alcényle en C 3-C 4, 2-propynyle, 3-halogéno-2-propynyle, phényle, un groupe phényle substitué par le fluor, le chlore, le brome, des groupes méthyle, méthoxy, nitro et/ou $CF_3$, un groupe benzyle ou un groupe benzyle substitué par le fluor, le chlore, le brome, des groupes méthyle, méthoxy, $NO_2$ et/ou $CF_3$ ; y compris leurs sels formés par addition avec des acides.

3. Composés de formule I selon la revendication 2, dans lesquels R représente l'hydrogène ; $R_1$ et $R_2$ représentent chacun, indépendamment l'un de l'autre, l'hydrogène, un groupe méthyle, le fluor, le chlore, le brome ou un groupe méthoxy ; $R_3$, $R_4$ et $R_5$ représentent chacun, indépendamment les uns des autres, l'hydrogène, le fluor, le chlore, le brome, un groupe $CF_3$, $NO_2$, méthyle ou méthoxy ; U et V forment ensemble le pont alkylène

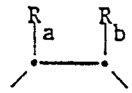

dans lequel $R_a$ représente l'hydrogène ou un groupe méthyle ; $R_b$ représente un groupe alkyle en C 1-C 4, alkyle en C 1-C 4 substitué par le fluor, le chlore ou le brome, phényle, phényle mono- à tri-substitué par le fluor, le chlore, le brome et/ou des groupes méthyle, ou le groupe $—CH_2—Z—R_h$ ; Z représente l'oxygène ou le soufre ; $R_h$ représente l'hydrogène, un groupe alkyle en C 1-C 4, un groupe alkyle en C 1-C 2 substitué par un groupe alcoxy en C 1-C 4, un groupe alcényle en C 3-C 4, 2-propynyle, 3-halogéno-2-propynyle, phényle, phényle substitué par le fluor, le chlore, le brome, des groupes méthyle, méthoxy, nitro et/ou $CF_3$, benzyle ou benzyle substitué par le fluor, le chlore, le brome, des groupes méthyle, méthoxy, nitro et/ou $CF_3$.

4. Composés de formule I selon la revendication 1, dans lesquels R représente l'hydrogène ou un groupe méthyle ; $R_1$ et $R_2$ représentent chacun, indépendamment l'un de l'autre, l'hydrogène, un groupe méthyle, éthyle, le fluor, le chlore, le brome ou un groupe méthoxy ; $R_3$, $R_4$ et $R_5$ représentent chacun, indépendamment les uns des autres, l'hydrogène, le fluor, le chlore, le brome, un groupe $CF_3$, $NO_2$, méthyle ou méthoxy ; U et V représentent chacun, indépendamment l'un de l'autre, un groupe alkyle en C 1-C 3, ou forment ensemble le pont alkylène

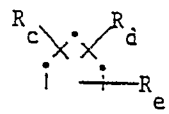

dans lequel $R_c$, $R_d$ et $R_e$ représentent chacun, indépendamment les uns des autres, l'hydrogène ou un groupe alkyle en C 1-C 4 et la somme des atomes de carbone de $R_c$, $R_d$ et $R_e$ ne dépasse pas 6 ; y compris leurs sels formés par addition avec des acides.

5. Composés de formule I selon la revendication 4, dans lesquels R représente l'hydrogène ; $R_1$ et $R_2$ représentent chacun, indépendamment l'un de l'autre, l'hydrogène, un groupe méthyle, le fluor, le chlore, le brome ou un groupe méthoxy ; $R_3$, $R_4$ et $R_5$ représentent chacun, indépendamment les uns des autres, l'hydrogène, le fluor, le chlore, le brome, un groupe $CF_3$, $NO_2$, méthyle ou méthoxy ; U et V forment ensemble le pont alkylène

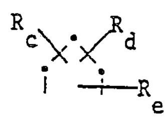

dans lequel $R_c$ représente l'hydrogène et $R_d$ et $R_e$ représentent chacun, indépendamment l'un de l'autre, l'hydrogène, un groupe méthyle ou éthyle.

6. Composés de formule I selon la revendication 1, dans lesquels R représente l'hydrogène ou un groupe méthyle ; $R_1$ et $R_2$ représentent chacun, indépendamment l'un de l'autre, l'hydrogène, un groupe méthyle, éthyle, le fluor, le chlore, le brome ou un groupe méthoxy ; $R_3$, $R_4$ et $R_5$ représentent chacun, indépendamment les uns des autres, l'hydrogène, le fluor, le chlore, le brome, un groupe $CF_3$, $NO_2$, méthyle ou méthoxy ; U et V représentent chacun, indépendamment l'un de l'autre, un groupe alkyle en C 1-C 3 ou forment ensemble le pont alkylène

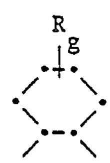

dans lequel $R_g$ représente l'hydrogène ou un groupe alkyle en C 1-C 4 ; y compris leurs sels formés par addition avec des acides.

7. Composés de formule I selon la revendication 6, dans lesquels R représente l'hydrogène ; $R_1$ et $R_2$ représentent chacun, indépendamment l'un de l'autre, l'hydrogène, un groupe méthyle, le fluor, le chlore, le brome ou un groupe méthoxy ; $R_3$, $R_4$ et $R_5$ représentent chacun, indépendamment les uns des autres, l'hydrogène, le fluor, le chlore, le brome, un groupe $CF_3$, $NO_2$, méthyle ou méthoxy ; U et V forment ensemble le pont alkylène

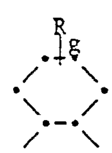

dans lequel $R_9$ représente l'hydrogène, un groupe méthyle ou éthyle.

8. Un composé de formule I selon la revendication 1, à l'état de mélange d'isomères ou à l'état d'isomère optique pur, choisi dans le groupe suivant :

3-[2-(2,4-dichlorobenzyl)-4-méthoxyméthyl-1,3-dioxolanne-2-yl]-pyridine,
3-[2-(2,4-dichlorobenzyl)-4-méthyl-1,3-dioxolanne-2-yl]-pyridine,
3-[2-(2,4-dichlorobenzyl)-4-chlorométhyl-1,3-dioxolanne-2-yl]-pyridine,
3-[2-(2,4-dichlorobenzyl)-1,3-dioxanne-2-yl]-pyridine,
3-[2-(2,4-dichlorobenzyl)-4-hydroxyméthyl-1,3-dioxolanne-2-yl]-pyridine,
3-[2-(2,4-dichlorobenzyl)-4-éthyl-1,3-dioxolanne-2-yl]-pyridine.

9. Procédé de préparation des composés de formule I, caractérisé

A) par une réaction d'acétalisation d'une cétone de formule II en présence d'un acide

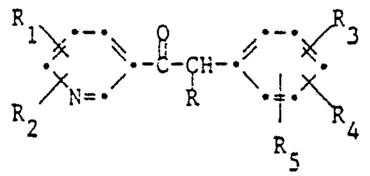

(II)

à l'aide d'un alcool V—OH ou U—OH, d'un diol de formule HO—U—V—OH ou d'un orthoester d'acide carboxylique R'—C(OV)$_3$ ou R'—C(OU)$_3$, ou par transacétalisation de l'acétal obtenu à l'aide d'un excès de l'alcanol ou diol correspondant, les symboles R à $R_5$, U et V de la formule II et des formules des alcools et diols ayant les significations indiquées en référence à la formule I et R' représentant de préférence un groupe alkyle inférieur ; ou bien

E) par la réaction d'un composé de formule I'

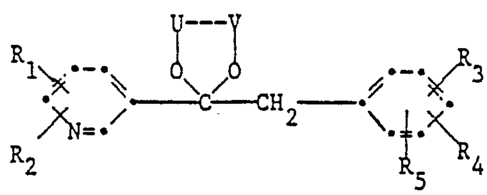

(I')

avec un composé de formule IX

R—X (IX)

les symboles R à $R_5$ et U et V ayant dans les formules I' et IX les significations indiquées en référence à la formule I et X représentant un groupe éliminable usuel.

10. Procédé de préparation des composés de formule I, caractérisé

B) par la réaction d'un composé de formule III

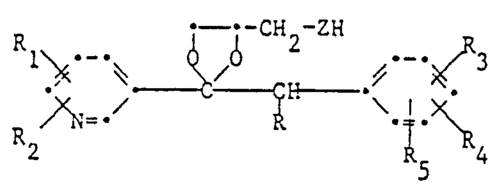

(III)

avec un composé réactif, convenant pour une O-alkylation ou une S-alkylation, qui répond à la formule IV

$R_h$—X (IV)

les symboles R à $R_5$, $R_h$ et Z des formules III et IV ayant les significations indiquées en référence à la formule I et X représentant l'un des groupes éliminables usuels ; ou bien

C) par la réaction d'un acétal de formule V

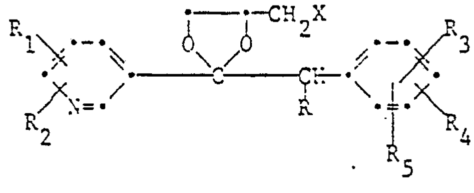

(V)

avec un composé de formule VI

$R_h$—Z—M (VI)

dans laquelle les symboles R à $R_5$, $R_h$ et Z ont les significations indiquées en référence à la formule I, X a les significations indiquées dans la variante B et M représente l'hydrogène ou, de préférence, un atome métallique, en particulier un atome de métal alcalin ; ou bien

D) par une réaction de condensation d'un alcool de formule VII avec un alcanol de formule VIII

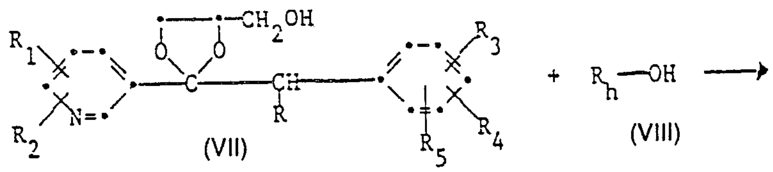
(VII) (VIII)
(I)

les symboles R à $R_5$ et $R_h$ ayant les significations indiquées en référence à la formule I.

11. Produit microbicide pour combattre ou prévenir une attaque par des microorganismes, caractérisé en ce qu'il contient, avec des additifs usuels, au moins un composant actif consistant en un composé de formule I selon la revendication 1.

12. Produit selon la revendication 10, caractérisé en ce qu'il contient au moins un composant actif consistant en un composé de formule I selon la revendication 2.

13. Produit pour combattre les microorganismes selon la revendication 10, caractérisé en ce qu'il contient au moins un composant actif consistant en un composé de formule I selon la revendication 3.

14. Produit pour combattre les microorganismes selon la revendication 10, caractérisé en ce qu'il contient au moins un composant actif consistant en un composé de formule I selon l'une des revendications 4 à 8.

15. Procédé pour combattre ou prévenir une attaque de végétaux cultivés par des microorganismes phytopathogènes, caractérisé en ce que l'on applique sur la plante ou son habitat un composé de formule I selon l'une des revendications 1 à 8.

**Revendications** (pour l'Etat contractant AT)

1. Produit microbicide pour combattre une attaque par des microorganismes, caractérisé en ce qu'il contient, avec des additifs usuels, au moins un composant actif consistant en un composé de formule I

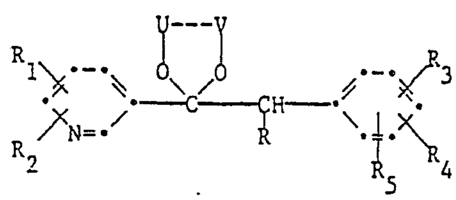
(I)

dans laquelle

R représente l'hydrogène, un groupe alkyle en C 1-C 6, cycloalkyle en C 3-C 6, phényle, benzyle, phényle substitué par des groupes alkyle en C 1-C 4, des halogènes, des groupes alcoxy en C 1-C 4, $NO_2$ et/ou $CF_3$, ou benzyle substitué par des groupes alkyle en C 1-C 4, des halogènes, des groupes alcoxy en C 1-C 4, $NO_2$ et/ou $CF_3$ ;

$R_1$ et $R_2$ représentent chacun, indépendamment l'un de l'autre, l'hydrogène, un groupe alkyle en C 1-C 4, un halogène ou un groupe alcoxy en C 1-C 4 ;

$R_3$, $R_4$ et $R_5$ représentent chacun, indépendamment les uns des autres, l'hydrogène, un halogène, un groupe $CF_3$, $NO_2$, alkyle en C 1-C 4, alcoxy en C 1-C 4, phényle ou phényle substitué par des groupes alkyle en C 1-C 4, alcoxy en C 1-C 4, des halogènes, des groupes $NO_2$ et/ou $CF_3$ ;

U et V représentent chacun, indépendamment l'un de l'autre, un groupe alkyle en C 1-C 6 ou un groupe alkyle substitué par des halogènes ou des groupes alcoxy en C 1-C 4 ou forment ensemble l'un des ponts alkylène suivants

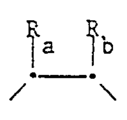 , 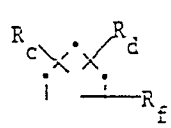 ou 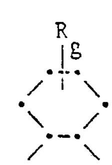

dans lesquels

$R_a$ représente l'hydrogène, un groupe alkyle en C 1-C 12, un groupe alkyle en C 1-C 12 mono- ou poly-substitué par des halogènes, un groupe phényle, un groupe phényle mono- ou poly-substitué par des halogènes et/ou des groupes alkyle en C 1-C 4, ou le groupe $—CH_2—Z—R_h$ ;

$R_b$ représente un groupe alkyle en C 1-C 12, un groupe alkyle en C 1-C 12 mono- ou poly-substitué par des halogènes, un groupe phényle, un groupe phényle mono- ou poly-substitué par des halogènes et/ou des groupes alkyle en C 1-C 4, ou le groupe $—CH_2—Z—R_h$ ;

Z représente l'oxygène ou le soufre,

$R_h$ représente l'hydrogène, un groupe alkyle en C 1-C 8, un groupe alkyle en C 1-C 8 substitué par des groupes alcoxy en C 1-C 4, un groupe alcényle en C 3-C 4, 2-propynyle, 3-halogéno-2-propynyle, phényle, un groupe phényle substitué par des halogènes, des groupes alkyle en C 1-C 4, alcoxy en C 1-C 4, $NO_2$ et/ou $CF_3$, un groupe benzyle ou un groupe benzyle substitué par des halogènes, des groupes alkyle en C 1-C 4, alcoxy en C 1-C 4, $NO_2$ et/ou $CF_3$ ;

$R_c$, $R_d$ et $R_f$ représentent chacun, indépendamment les uns des autres, l'hydrogène ou un groupe alkyle en C 1-C 4 ; et

$R_g$ représente l'hydrogène ou un groupe alkyle en C 1-C 4 ;

y compris leurs sels formés par addition avec des acides.

2. Produit selon la revendication 1, contenant un composé de formule I dans laquelle R représente l'hydrogène ou un groupe méthyle ; $R_1$ et $R_2$ représentent chacun, indépendamment l'un de l'autre, l'hydrogène, un groupe méthyle, éthyle, le fluor, le chlore, le brome ou un groupe méthoxy ; $R_3$, $R_4$ et $R_5$ représentent chacun, indépendamment les uns des autres, l'hydrogène, le fluor, le chlore, le brome, un groupe $CF_3$, $NO_2$, méthyle ou méthoxy ; U et V représentent chacun, indépendamment l'un de l'autre, un groupe alkyle en C 1-C 3 ou forment ensemble un pont alkylène

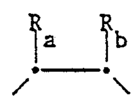

dans lequel $R_a$ représente l'hydrogène, un groupe alkyle en C 1-C 4, un groupe phényle, un groupe phényle mono- à tri-substitué par le fluor, le chlore, le brome et/ou des groupes méthyle, ou le groupe $—CH_2—Z—R_h$ ; $R_b$ représente un groupe alkyle en C 1-C 4, un groupe alkyle en C 1-C 4 mono- à tri-substitué par le fluor, le chlore et/ou le brome, un groupe phényle, un groupe phényle mono- à tri-substitué par le fluor, le chlore, le brome et/ou des groupes méthyle ; ou le groupe $—CH_2—Z—R_h$ ; Z représente l'oxygène ou le soufre ; $R_h$ représente l'hydrogène, un groupe alkyle en C 1-C 6, un groupe alkyle en C 1-C 3 substitué par des groupes alcoxy en C 1-C 4, un groupe alcényle en C 3-C 4, 2-propynyle, 3-halogéno-2-propynyle, phényle, un groupe phényle substitué par le fluor, le chlore, le brome, des groupes méthyle, méthoxy, nitro et/ou $CF_3$, un groupe benzyle ou un groupe benzyle substitué par le fluor, le chlore, le brome, des groupes méthyle, méthoxy, $NO_2$ et/ou $CF_3$ ; y compris leurs sels formés par addition avec des acides.

3. Produit selon la revendication 2, contenant un composé de formule I dans laquelle R représente l'hydrogène ; $R_1$ et $R_2$ représentent chacun, indépendamment l'un de l'autre, l'hydrogène, un groupe méthyle, le fluor, le chlore, le brome ou un groupe méthoxy ; $R_3$, $R_4$ et $R_5$ représentent chacun, indépendamment les uns des autres, l'hydrogène, le fluor, le chlore, le brome, un groupe $CF_3$, $NO_2$, méthyle ou méthoxy ; U et V forment ensemble le pont alkylène

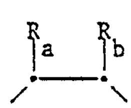

dans lequel $R_a$ représente l'hydrogène ou un groupe méthyle ; $R_b$ représente un groupe alkyle en C 1-C 4, alkyle en C 1-C 4 substitué par le fluor, le chlore ou le brome, phényle, phényle mono- à tri-substitué par le fluor, le chlore, le brome et/ou des groupes méthyle, ou le groupe $—CH_2—Z—R_h$ ; Z représente l'oxygène ou le soufre ; $R_h$ représente l'hydrogène, un groupe alkyle en C 1-C 4, un groupe alkyle en C 1-C 2 substitué par un groupe alcoxy en C 1-C 4, un groupe alcényle en C 3-C 4, 2-propynyle, 3-halogéno-2-propynyle, phényle, phényle substitué par le fluor, le chlore, le brome, des groupes méthyle, méthoxy, nitro et/ou $CF_3$, benzyle ou benzyle substitué par le fluor, le chlore, le brome, des groupes méthyle, méthoxy, nitro et/ou $CF_3$.

4. Produit selon la revendication 1, contenant un composé de formule I dans laquelle R représente l'hydrogène ou un groupe méthyle ; $R_1$ et $R_2$ représentent chacun, indépendamment l'un de l'autre, l'hydrogène, un groupe méthyle, éthyle, le fluor, le chlore, le brome ou un groupe méthoxy ; $R_3$, $R_4$ et $R_5$ représentent chacun, indépendamment les uns des autres, l'hydrogène, le fluor, le chlore, le brome, un groupe $CF_3$, $NO_2$, méthyle ou méthoxy ; U et V représentent chacun, indépendamment l'un de l'autre, un groupe alkyle en C 1-C 3, ou forment ensemble le pont alkylène

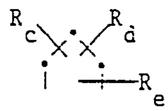

dans lequel $R_c$, $R_d$ et $R_e$ représentent chacun, indépendamment les uns des autres, l'hydrogène ou un groupe alkyle en C 1-C 4 et la somme des atomes de carbone de $R_c$, $R_d$ et $R_e$ ne dépasse pas 6 ; y compris leurs sels formés par addition avec des acides.

5. Produit selon la revendication 4, contenant un composé de formule I dans laquelle R représente l'hydrogène ; $R_1$ et $R_2$ représentent chacun, indépendamment l'un de l'autre, l'hydrogène, un groupe méthyle, le fluor, le chlore, le brome ou un groupe méthoxy ; $R_3$, $R_4$ et $R_5$ représentent chacun, indépendamment les uns des autres, l'hydrogène, le fluor, le chlore, le brome, un groupe $CF_3$, $NO_2$, méthyle ou méthoxy ; U et V forment ensemble le pont alkylène

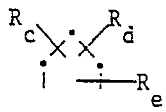

dans lequel $R_c$ représente l'hydrogène et $R_d$ et $R_e$ représentent chacun, indépendamment l'un de l'autre, l'hydrogène, un groupe méthyle ou éthyle.

6. Produit selon la revendication 1, contenant un composé de formule I dans laquelle R représente l'hydrogène ou un groupe méthyle ; $R_1$ et $R_2$ représentent chacun, indépendamment l'un de l'autre, l'hydrogène, un groupe méthyle, éthyle, le fluor, le chlore, le brome ou un groupe méthoxy ; $R_3$, $R_4$ et $R_5$ représentent chacun, indépendamment les uns des autres, l'hydrogène, le fluor, le chlore, le brome, un groupe $CF_3$, $NO_2$, méthyle ou méthoxy ; U et V représentent chacun, indépendamment l'un de l'autre, un groupe alkyle en C 1-C 3 ou forment ensemble le pont alkylène

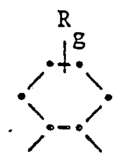

dans lequel $R_g$ représente l'hydrogène ou un groupe alkyle en C 1-C 4 ; y compris leurs sels formés par addition avec des acides.

7. Produit selon la revendication 6, contenant un composé de formule I dans laquelle R représente l'hydrogène ; $R_1$ et $R_2$ représentent chacun, indépendamment l'un de l'autre, l'hydrogène, un groupe méthyle, le fluor, le chlore, le brome ou un groupe méthoxy ; $R_3$, $R_4$ et $R_5$ représentent chacun, indépendamment les uns des autres, l'hydrogène, le fluor, le chlore, le brome, un groupe $CF_3$, $NO_2$, méthyle ou méthoxy ; U et V forment ensemble le pont alkylène

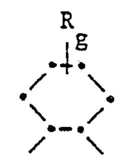

dans lequel $R_g$ représente l'hydrogène, un groupe méthyle ou éthyle.

8. Produit selon la revendication 1, contenant un composé de formule I, à l'état de mélange d'isomères ou à l'état d'isomère optique pur, choisi dans le groupe suivant :

3-[2-(2,4-dichlorobenzyl)-4-méthoxyméthyl-1,3-dioxolanne-2-yl]-pyridine,
3-[2-(2,4-dichlorobenzyl)-4-méthyl-1,3-dioxolanne-2-yl]-pyridine,
3-[2-(2,4-dichlorobenzyl)-4-chlorométhyl-1,3-dioxolanne-2-yl]-pyridine,
3-[2-(2,4-dichlorobenzyl)-1,3-dioxanne-2-yl]-pyridine,
3-[2-(2,4-dichlorobenzyl)-4-hydroxyméthyl-1,3-dioxolanne-2-yl]-pyridine,
3-[2-(2,4-dichlorobenzyl)-4-éthyl-1,3-dioxolanne-2-yl]-pyridine.

9. Procédé de préparation des composés de formule I, caractérisé

A) par une réaction d'acétalisation d'une cétone de formule II en présence d'un acide

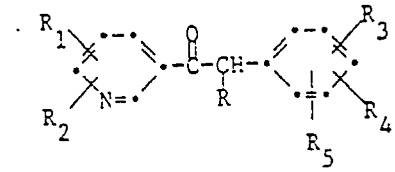

(II)

à l'aide d'un alcool V—OH ou U—OH, d'un diol de formule HO—U—V—OH ou d'un orthoester d'acide carboxylique R'—C(OV)$_3$ ou R'—C(OU)$_3$, ou par transacétalisation de l'acétal obtenu à l'aide d'un excès de l'alcanol ou diol correspondant, les symboles R à $R_5$, U et V de la formule II et des formules des alcools et diols ayant les significations indiquées en référence à la formule I et R' représentant de préférence un groupe alkyle inférieur ; ou bien

E) par la réaction d'un composé de formule I'

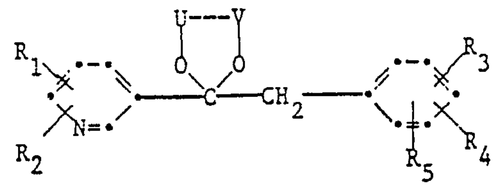

(I')

avec un composé de formule IX

R—X (IX)

les symboles R à $R_5$ et U et V ayant dans les formules I' et IX les significations indiquées en référence à la formule I et X représentant un groupe éliminable usuel.

10. Procédé de préparation des composés de formule I, caractérisé

B) par la réaction d'un composé de formule III

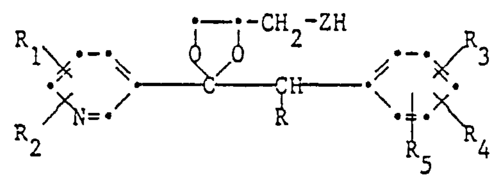

(III)

avec un composé réactif, convenant pour une O-alkylation ou une S-alkylation, qui répond à la formule IV

$R_h$—X (IV)

les symboles R à $R_5$, $R_h$ et Z des formules III et IV ayant les significations indiquées en référence à la formule I et X représentant l'un des groupes éliminables usuels ; ou bien

C) par la réaction d'un acétal de formule V

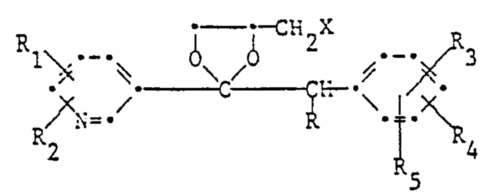

(V)

avec un composé de formule VI

$R_h$—Z—M (VI)

dans laquelle les symboles R à $R_5$, $R_h$ et Z ont les significations indiquées en référence à la formule I, X a les significations indiquées dans la variante B et M représente l'hydrogène ou, de préférence, un atome métallique, en particulier un atome de métal alcalin ; ou bien

D) par une réaction de condensation d'un alcool de formule VII avec un alcanol de formule VIII

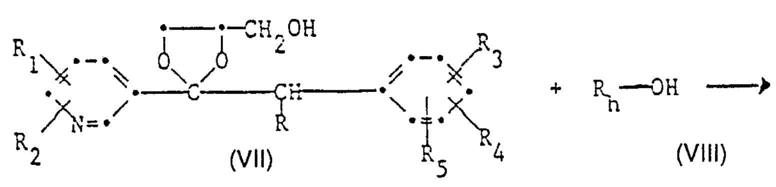

(VII) (VIII) (I)

les symboles R à $R_5$ et $R_h$ ayant les significations indiquées en référence à la formule I.

11. Procédé de préparation d'un produit agro-chimique, caractérisé en ce que l'on mélange

intimement au moins un composé de formule I avec des additifs appropriés solides ou liquides et des agents tensioactifs.

12. Procédé pour combattre ou prévenir une attaque de végétaux cultivés par des microorganismes phytopathogènes, caractérisé en ce que l'on applique sur la plante ou son habitat un composé de formule I.